(19) Europäisches Patentamt
European Patent Office
Office européen des brevets

(11) EP 0 411 733 B1

(12) EUROPÄISCHE PATENTSCHRIFT

(45) Veröffentlichungstag und Bekanntmachung des Hinweises auf die Patenterteilung:
**21.10.1998 Patentblatt 1998/43**

(51) Int Cl.6: **C07J 1/00**, C07J 41/00, A61K 31/56

(21) Anmeldenummer: **90250199.8**

(22) Anmeldetag: **06.08.1990**

# (54) 11 Beta-Aryl-gona-4,9-dien-3-one

11-Beta-aryle-gona-4,9-dien-3-ones

11-Bêta-aryl-gona-4,9-diène-3-ones

(84) Benannte Vertragsstaaten:
**AT BE CH DE DK ES FR GB GR IT LI LU NL SE**

(30) Priorität: **04.08.1989 DD 331479**
**16.08.1989 DD 331818**
**09.10.1989 DD 333409**

(43) Veröffentlichungstag der Anmeldung:
**06.02.1991 Patentblatt 1991/06**

(73) Patentinhaber: **SCHERING AKTIENGESELLSCHAFT**
**13342 Berlin (DE)**

(72) Erfinder:
- **Kasch, Helmut, Dr.**
  **DDR-6902 Jena (DD)**
- **Bertram, Gudrun**
  **O-6551 Haidefeld 22 (DE)**
- **Ponsold, Kurt, Prof.Dr.**
  **DDR-6900 Jena (DD)**
- **Schubert, Gerd, Dr.**
  **DDR-6900 Jena (DD)**
- **Röhrig, Heidemarie**
  **DDR-6900 Jena (DD)**
- **Kurischko, Anatoli**
  **DDR-6900 Jena (DD)**
- **Menzenbach, Bernd, Dr.**
  **DDR-6900 Jena (DD)**

(56) Entgegenhaltungen:

| | |
|---|---|
| **EP-A- 0 097 572** | **EP-A- 0 104 387** |
| **EP-A- 0 110 434** | **EP-A- 0 129 499** |
| **EP-A- 0 135 400** | **EP-A- 0 147 361** |
| **EP-A- 0 184 471** | **EP-A- 0 190 759** |
| **EP-A- 0 254 670** | **EP-A- 0 277 676** |
| **EP-A- 0 289 073** | **EP-A- 0 299 913** |
| **EP-A- 0 305 242** | **EP-A- 0 308 345** |
| **EP-A- 0 321 010** | **WO-A-83/03099** |
| **WO-A-89/12448** | **FR-A- 2 586 021** |
| **GB-A- 2 160 873** | |

- **Fertilität (1992) 8, 167-170**

Bemerkungen:
Die Akte enthält technische Angaben, die nach dem Eingang der Anmeldung eingereicht wurden und die nicht in dieser Patentschrift enthalten sind.

EP 0 411 733 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft 11β-Aryl-gona-4,9-diene der allgemeinen Formel I

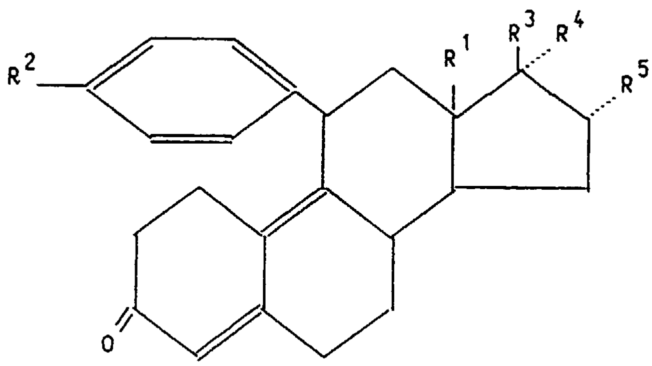

(I)

worin

A)

$R^1$ eine Methyl- oder Ethylgruppe,
$R^2$ eine Alkoxy-, Alkylthio-, wobei unter Alkyl eine Alkyl-, Alkenyl- oder eine entsprechende cyclische Verbindung mit 1 bzw. 2 bzw. 3 bis 7 Kohlenstoffatomen zu verstehen ist, eine Dimethylamino-, Monomethylamino-, Cyano-, Formyl-, Acetyl-, oder 1-Hydroxyethylgruppe,
$R^3$ eine Alkoxymethoxy-, oder Alkoxygruppe mit jeweils 1 bis 6 Kohlenstoffatomen, und
$R^4$ eine Ethinyl-, Prop-1-inyl, 3-Hydroxyprop-1-inyl-, 3-Alkanoyloxyprop-1-inyl-, 3-Alkanoyloxyprop-1-enyl-, 3-Alkanoyloxypropylgruppe mit jeweils 1 bis 6 Kohlenstoffatomen, 3-Hydroxyprop-1-enyl- und 3-Hydroxypropylgruppe sowie
$R^5$ ein Wasserstoffatom

oder
B)

| | |
|---|---|
| $R^1$ | eine Methyl- oder Ethylgruppe, |
| $R^2$ | eine Methoxy-, Thiomethyl-, Dimethylamino-, Monomethylamino-, Cyano-, Formyl-, Acetyl- odere 1-Alkoxyethylgruppe mit 1 bis 6 Kohlenstoffatomen im Alkoxyrest, |
| $R^3$ | eine Alkoxy-, Alkoxymethyloxy-, Alkanoyloxyethyloxy-, mit jeweils 1 bis 6 Kohlenstoffatomen im Alkoxyrest, und |
| $R^4$ | eine Ethinyl-, Prop-1-inyl-, Alkyl-, 3-Alkoxyprop-1-inyl-, 3-Alkoxypropylgruppe mit jeweils 1 bis 4 Kohlenstoffatomen im Alkyl- bzw. Alkoxyrest oder die Gruppierung $-CH_2Y$, worin Y ein Cyano-, Azido- oder Alkoxyrest mit 1 bis 6 Kohlenstoffatomen sowie |
| $R^5$ | ein Wasserstoffatom oder eine Alkylgruppe mit 1 bis 4 Kohlenstoffatomen oder aber |
| $R^4$ und $R^5$ | gemeinsam eine Tetramethylenbrücke |

oder
C)

$R^1$ eine Methylgruppe,
$R^2$ eine Dimethylamino, eine freie oder ketalisierte Acetylgruppe,
$R^3$ eine Alkoxygruppe mit 1 bis 6 Kohlenstoffatomen oder Alkylthiomethyloxygruppe mit 1 bis 4 Kohlenstoffatomen im Alkylrest,
$R^4$ eine Alkoxymethylgruppe mit 1 bis 6 Kohlenstoffatomen im Alkoxyrest sowie
$R^5$ ein Wasserstoffatom

bedeutet.

Vorzugsweise bedeutet dabei

$R^1$ eine Methylgruppe,
$R^2$ eine Dimethylamino- oder Acetylgruppe und außerdem

in A)

$R^3$ eine Methoxygruppe,
$R^4$ eine Prop-1-inyl-, Ethinyl-, 3-Hydroxy-prop-1-inyl-, 3-Hydroxy-prop-1(Z)-enyl-, 3-Hydroxypropylgruppe und
$R^5$ ein Wasserstoffatom,

in B)

$R^3$ eine Methoxygruppe,
$R^4$ eine Prop-1-inyl-, Ethinyl- oder 3-Hydroxypropylgruppe,
$R^5$ ein Wasserstoffatom oder
$R^4$ und $R^5$ gemeinsam eine Tetramethylenbrücke,

in C)

$R^3$ eine Methoxygruppe,
$R^4$ eine Methoxymethyl-, Ethoxymethyl- oder Propoxymethylgruppe und
$R^5$ ein Wasserstoff.

Insbesondere bevorzugt sind folgende Verbindungen der allgemeinen Formel I

11β-(4-Dimethylaminophenyl)-17β-methoxy-17α-propinyl-13-ethyl-gona-4,9-dien-3-on,
11β-(4-Acetylphenyl)-17β-methoxy-17α-propinyl-13-methyl-gona-4,9-dien-3-on,
11β-(4-Dimethylaminophenyl)-17β-methoxy-17α-ethinyl-13-methyl-gona-4,9-dien-3-on,
11β-(4-Acetylphenyl)-17β-methoxy-17α-ethinyl-13-methyl-gona-4,9-dien-3-on,
11β-(4-Acetylphenyl)-17α-(3'-hydroxy-1-propinyl)-17β-methoxy-13-methyl-gona-4,9-dien-3-on,
11β-(4-Acetylphenyl)-17α-(3'-hydroxy-1'(Z)-propenyl)-17β-methoxy-13-methyl-gona-4,9-dien-3-on sowie
11β-(4-Acetylphenyl)-17α-(3'-hydroxypropyl)-17β-methoxy-13-methyl-gona-4,9-dien-3-on,
11β-(4-Acetylphenyl)-17β-methoxy-17α-methoxymethyl-estra-4,9-dien-3-on,
11β-(4-Dimethylaminophenyl)-17β-methoxy-17α-methoxymethyl-estra-4,9-dien-3-on,
11β-(4-Acetylphenyl)-17α-ethoxymethyl-17β-methoxy-estra-4,9-dien-3-on,
11β-(4-Acetylphenyl)-17β-methoxy-17α-propoxymethyl-estra-4,9-dien-3-on,
11β-(4-Dimethylaminophenyl)-17β-methoxy-17α-propinyl-13-methyl-gona-4,9-dien-3-on,
11β-(4-Methoxyphenyl)-17β-methoxy-17α-propinyl-13-methyl-gona-4,9-dien-3-on,
11β-(4-Dimethylaminophenyl)-17α-(3'-hydroxy-1'-Z-propenyl)-17β-methoxy-13-methyl-gona-4,9-dien-3-on,
11β-(4-Methoxyphenyl)-17β-methoxy-17α-(3'-hydroxy-1'-Z-propenyl)-13-methyl-gona-4,9-dien-3-on,
11β-(4-Dimethylaminophenyl)-17α-(3'-hydroxypropyl)-17β-methoxy-13-methyl-gona-4,9-dien-3-on,
11β-(4-Methoxyphenyl)-17α-(3'-hydroxypropyl)-17β-methoxy-13-methyl-gona-4,9-dien-3-on,
11β-(4-Dimethylaminophenyl)-17α-(3'-hydroxy-1'-propinyl)-17β-methoxy-13-methyl-gona-4,9-dien-3-on,
11β-(4-Methoxyphenyl)-17α-(3'-hydroxy-1'-propinyl)-17β-methoxy-13-methyl-gona-4,9-dien-3-on,
11β-(4-Dimethylaminophenyl)-17α-ethinyl-17β-methoxy-13-methyl-gona-4,9-dien-3-on,
11β-(4-Methoxyphenyl)-17α-ethinyl-17β-methoxy-13-methyl-gona-4,9-dien-3-on,
11β-(4-Methoxyphenyl)-17β-methoxy-17α-methoxymethyl-13-methyl-gona-4,9-dien-3-on.

Die Verbindungen der allgemeinen Formel I besitzen eine starke Affinität zum Gestagenrezeptor, ohne selbst gestagene Wirkung zu entfalten. Sie sind kompetitive Antagonisten des Progesterons (Antigestagene); da sie das zur Aufrechterhaltung der Schwangerschaft erforderliche Progesteron vom Rezeptor verdrängen, sind sie zur Auslösung von Aborten und zur Einleitung der Geburt geeignet.

Neben den genannten Indikationen können die erfindungsgemäßen Verbindungen auch zur Behandlung der Endometriose, Dysmenorrhoe und endokriner hormonabhängiger Tumore, wie z.B. Mamma-Carcinom und Meningeom verwendet werden.

Zur Kennzeichnung der antigestagenen Wirkung dient die tierexperimentell ermittelt abortive Wirkung der Substanzen. Zu diesem Zweck wurden weibliche gravide Raten (positiver Spermiennachweis = 1 Graviditätstag) im Gewicht zwischen 180 und 200 g am 5. bis 8. Graviditätstag mit der Testverbindung in Erdnußöl suspendiert subkutan behandelt. Nach Autopsie am 20. Graviditätstag wurden die Uterie untersucht. Dabei wurde die Anzahl der graviden Weibchen und die durchschnittliche Zahl der Feten pro gravides Tier festgestellt. Der Hemmeffekt wurde wie folgt berechnet:

$$He = \left(1 - \frac{x_v \cdot n_k}{m_v \cdot x_k}\right) \cdot 100\ (\%)$$

x = Anzahl der graviden Weibchen
n = Anzahl der besamten Weibchen
v = Versuchsgruppe
k = Kontrollgruppe

| Gruppe Substans | Gesamtdosis (mg/Tier/4d) | N | Fertilitätshemmung | |
|---|---|---|---|---|
| | | | absol. | rel. % |
| 17β-Methoxy-17αMethoxymethyl-11β-acetylphenyl | 2 | 6 | 6 | 100 |
| 17β-Methoxy-17α-propinyl-11β-acetylphenyl | 2 | 6 | 6 | 100 |
| 17β-Methoxy-16α,17α-methylen 11β-acetylphenyl | 2 | 6 | 6 | 100 |
| 17β-Methoxy-17α-propinyl-11β-dimethylaminophenyl | 2 | 6 | 6 | 100 |
| 17β-Methoxy-17α-ethinyl-11β-acetylphenyl | 2 | 6 | 6 | 100 |
| Kontrolle | 0 | 6 | 0 | 0 |

Als Vergleich wurde die abortive Wirkung von RU 486 (11β-(4-Dimethylaminophenyl)-17β-hydroxy-17α-(prop-1-inyl)-4,9-estradien-3-on; EP-A-0057115) bestimmt: Sie weist im beschriebenen Test, ebenfalls bei einer Gesamtdosis von 2 mg, lediglich eine relative Fertilitätshemmung von 80 % auf.

Die Testung auf estrogene und gonadotrope Wirkung erfolgte im kolpotropen Test. Dabei wurde bei den beanspruchten Verbindungen keine Wirkung festgestellt

Die erfindungsgemäßen Verbindungen der allgemeinen Formel I können in Form pharmazeutischer Präparate Verwendung finden. Die Herstellung der Präparate erfolgt nach an sich bekannten Methoden der Galenik durch Mischen mit organischem inertem Trägermaterial, welches für die enterale, perkutane oder parenterale Applikation geeignet ist.

Die Dosierung der erfindungsgemäßen Verbindungen für die angegebenen Indikationen Liegt zwischen 1 und 1000 mg täglich.

Die Verbindungen der allgemeinen Formel I werden erfindungsgemäß hergestellt, indem eine Verbindung der allgemeinen Formel II

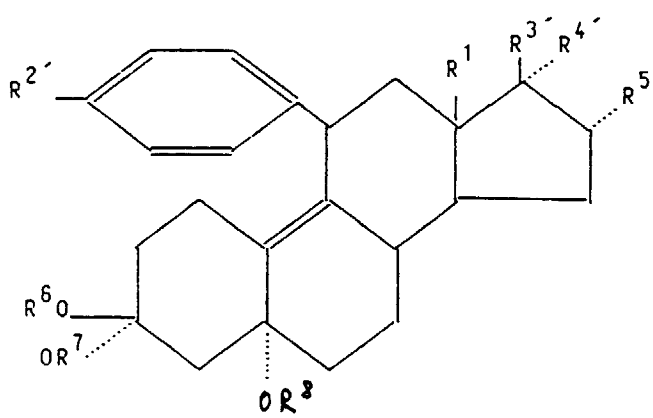

(II),

worin

$R^1$ und $R^5$ die in Formel I angegebene Bedeutung haben,
$R^{2'}$, $R^{3'}$ und $R^{4'}$ dieselbe Bedeutung wie $R^2$, $R^3$ und $R^4$ in Formel I haben, wobei gegebenenfalls vorhandene Keto- und/oder Hydroxygruppen geschützt sein können,
$R^6$ und $R^7$ je eine Methyl- oder Ethylgruppe oder gemeinsam eine Ethylen-oder 2,2-Dialkylpropylengruppe, insbesondere eine 2,2-Dimethylpropylengruppe sowie
$R^8$ ein Wasserstoffatom oder einen Alkylrest mit 1 bis 6 Kohlenstoffatomen bedeuten,

durch Säurebehandlung in einem mit Wasser mischbaren Lösungsmittel, gegebenenfalls unter Erwärmen auf 60°-80°C, in eine Verbindung der allgemeinen Formel I überführt.

Als Säuren für die Säurebehandlung werden z.B. wäßrige Essigsäure, p-Toluolsulfonsäure oder Mineralsäuren wie Salzsäure, Schwefelsäure, Phosphorsäure oder Perchlorsäure und als Lösungsmittel wäßriges Methanol, Ethanol oder Aceton verwendet. Gegebenenfalls wird während der Säurebehandlung auf 60° bis 80°C erwärmt. Die saure Behandlung kann auch an einem sauren Träger, wie z.B. saurem Aluminiumoxid, durchgeführt werden.

Die Herstellung der erfindungsgemäß zu verwendenden Ausgangsprodukte der allgemeinen Formel II erfolgt je nach den letztendlich gewünschten Substituenten nach unterschiedlichen Verfahren.

Zur Synthese der Ausgangsprodukte der allgemeinen Formel II, die letztlich zu den Endprodukten der allgemeinen Formel I mit den unter A) genannten Substituenten führen, bedient man sich der im Schema I wiedergegebenen Syntheserarte.

Schema I

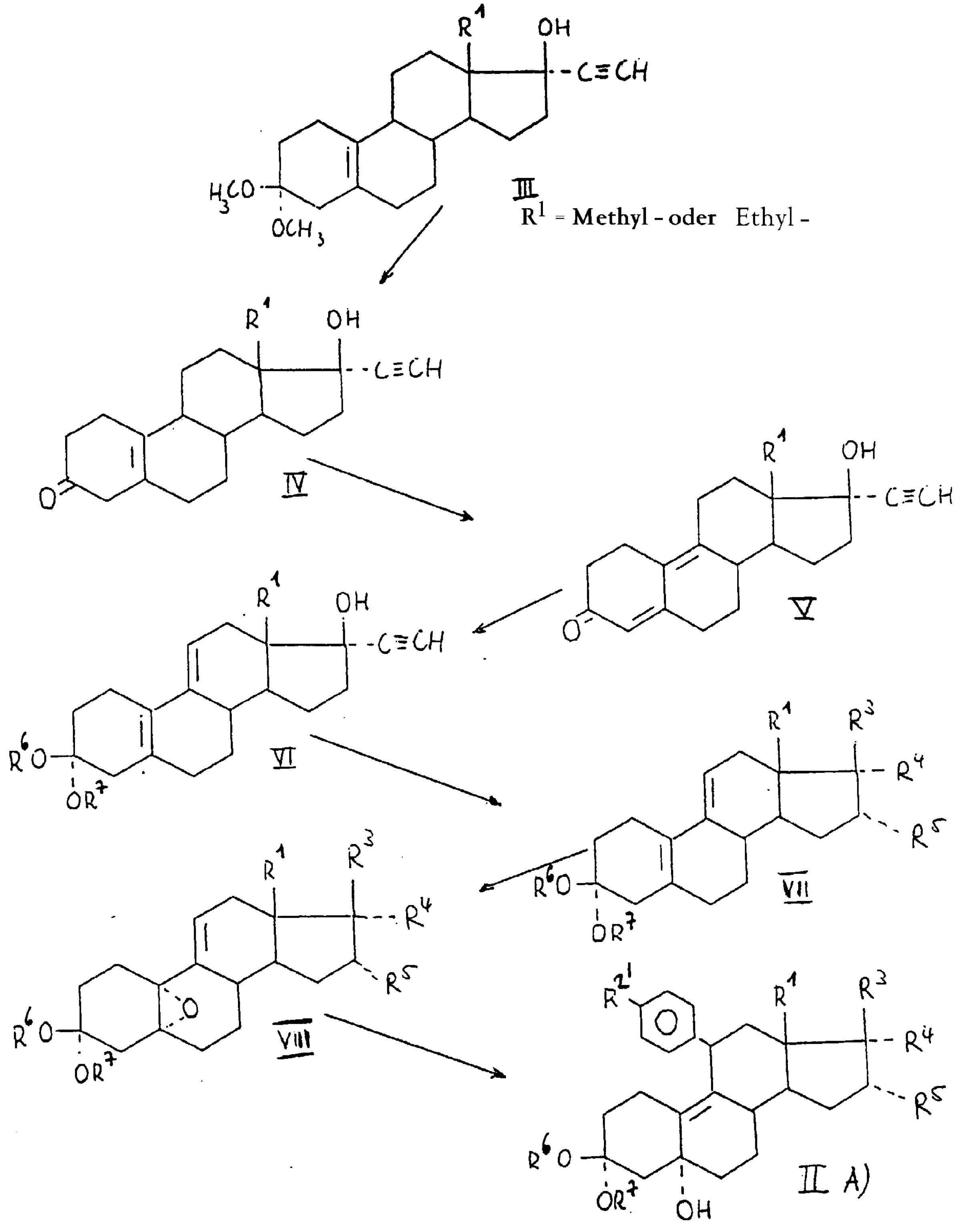


Dabei werden

a) 3,3-Dimethoxy-17α-ethinyl-gon-5(10)-en-17β-ole der allgemeinen Formel III durch Ketalspaltung in einem wasserhaltigen organischen Lösungsmittel in Gegenwart katalytischer Mengen Säure in 17α-Ethinyl-gon-5(10)-en-3-one der allgemeinen Formel IV umgewandelt,

b) die 17α-Ethinyl-gon-5(10)-en-3-one IV durch Bromierung/Dehydrobromierung in 17α-Ethinyl-17β-hydroxy-gona-4,9-dien-3-one der allgemeinen Formel V überführt,

c) aus den 17α-Ethinyl-17β-hydroxy-gona-4,9-dien-3-onen V durch Ketalisierung die Ketale der allgemeinen Formel VI herstellt, worin $R^7$ und $R^6$ = $CH_3$, $C_2H_5$ oder ein cyclisches Ketal mit 2 oder 3 C-Ringatomen, die an den Kohlenstoffatomen durch Alkylgruppen substituiert sein können, dargestellt,

d) die Ketale VI durch eine Base in einem Ether oder einem aprotisch dipolaren Lösungsmittel, gegebenenfalls unter Zusatz eines Lösungsvermittlers, in die Acetylide und/oder Alkoholate überführt, welche man mit einem Alkylierungsmittel wahlweise O und gegebenenfall C alkyliert oder durch Hydroxymethylierung der Ethinylgruppe mit Formaldehyd in Gona-5(10),9(11)-diene der allgemeinen Formel VII umwandelt,

e) aus den Gona-5(10),9(11)-dienen VII durch Epoxidierung die 5α,10α-Epoxide der allgemeinen Formel VIII synthetisiert und

f) die 5α,10α-Epoxide VIII mit Arylmagnesiumhalogenid in Gegenwart eines $Cu^I$-Salzes bei einer Reaktionstemperatur von -30°C bis +30°C reagieren läßt und Verbindungen mit einer 17α-Propargylgruppe reduziert, wobei 11β-Aryl-5α-hydroxy-gon-9-ene der allgemeinen Formel II A entstehen.

Bevorzugt wird im Verfahrensschritt a) als organisches Lösungsmittel Aceton, Methanol oder eine Lösungsmittelkombination, bestehend aus Methylenchlorid und tert.-Butanol, und als Säuren Oxalsäure, p-Toluolsulfonsäure oder Mineralsäuren, wie Schwefelsäure, Salzsäure, Perchlorsäure oder Phosphorsäure,

im Verfahrensschritt b)
für die Bromierung/Dehydrobromierung Pyridinhydrobromidperbromid oder Brom in Pyridin,

im Verfahrensschritt c)
für die Ketalisierung als Alkohole Methanol, Ethanol, Ethylenglykol oder 2,3-Dimethylpropandiol, als Säuren p-Toluolsulfonsäure, Oxalsäure, Pyridiniumtosylat oder Mineralsäuren, wie Schwefelsäure oder Perchlorsäure, als wasserentziehende Mittel Ameisensäuretriethyl- und -trimethylester oder ein Wasserschleppmittel, wie Chloroform, Benzol oder Toluol,

im Verfahrensschritt d)
als Basen Lithiumalkyle wie Lithiummethyl, -n-butyl und -tert.-butyl, Natriumhydrid, Kaliumhydroxid, Lithium-, Natrium- oder Kaliumaphthalid, oder durch elektrochemische Reduktion erzeugtes Naphthalidion, als Ether Diethylether, Tetrahydrofuran oder Dioxan, Dimethylsulfoxid, Dimethylformamid, als gegebenenfalls einzusetzender Lösungsvermittler Benzol oder Toluol und als Alkylierungsmittel Dialkylsulfate, wie Dimethyl- und Diethylsulfat, und Alkylhalogenide, wie Methyl-, Ethyl-, Propyl-, Butyl-, Pentyl-, Hexyl-, Isopropylhalogenide in Form der Chloride, Bromide und Yodide, sowie Methylchloralkylether mit einem Alkyl in der Bedeutung n = 1 bis 6,

im Verfahrensschritt e)
als Puffer $Na_2CO_3$, $NaHCO_3$, $K_2CO_3$, $KHCO_3$, $Na_2HPO_4$, $NaH_2PO_4$, NaOAc und KOAc sowie als inerte Lösungsmittel Benzol, Toluol, Chloroform, Dichlorethylen oder Methylenchlorid und

im Verfahrensschritt f)
als Arylmagnesiumhalogenide Phenylmagnesiumhalogenide in Form der Chloride und Bromide, die in p-Stellung zum Magnesium eine $OCH_3$, $SCH_3$, $N(CH_3)_2$, $NHCH_3$, CN, $CH_3CHOH$, $R_4O-CH-OR_5$, $CH_3-COR_4-OR_5$-Gruppe enthalten, wobei $R_5 = R_2 = CH_3$, $C_2H_5$ oder ein cyclisches Ketal mit 2 oder 3-C-Ringatomen, die an den Kohlenstoffatomen durch Alkylgruppen substituiert sein können, darstellt, als Ether Diethylether, Tetrahydrofuran und Dioxan, als Lösungsvermittler Benzol und Toluol, als $Cu^I$-Salze CuCN, CuI und CuCl sowie als Reduktionsmittel für die 17α-Propargylgruppe Lithiumalanat oder Wasserstoff in Gegenwart von Pd oder Pt als Hydrierkatalysator in einem Ether bzw. einem Alkohol verwendet.

In der besonders bevorzugten Ausführungsform des Verfahrens wird die Alkylierung der 17α-Ethinyl- und der 17β-Sauerstofffunktion bzw. die Hydroxymethylierung der 17α-Ethinylgruppe auch ausgehend von den Edukten gemäß Formel I durchgeführt.

Mit dem vorgeschlagenen Verfahren können ausgehend von einem technisch gut zugänglichen Ausgangsprodukt Verbindungen mit unterschiedlicher Substitution am C-Atom 17 mit relativ einfachen Roh- und Hilfsstoffen hergestellt werden. Auf diese Weise werden neben den 17α-Ethinyl-, die 17α-Propinyl-, 17α-Hydroxypropinyl- und auch gleichzeitig die diversen 17β-Alkylether zugänglich.

Zur Gewinnung der für die Endprodukte der allgemeinen Formel I mit den unter B) genannten Substituenten erforderlichen Ausgangsprodukte der allgemeinen Formel II wird eine zur Verbindung IIA) aus Schema 1 analoge Verbindung der allgemeinen Formel IIB)

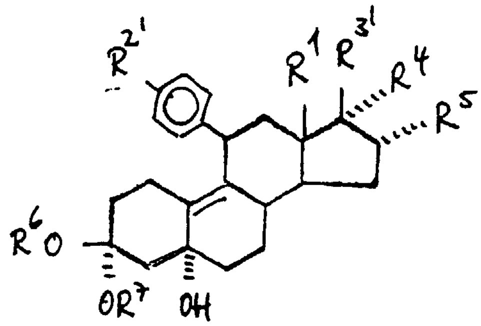

II B)

worin $R^{2'}$ dieselbe Bedeutung wie $R^2$ in Formel I unter B) hat, wobei eine gegebenenfalls vorhandene Carbonylgruppe als Ketal geschützt ist, und $R^1$, $R^3$, $R^4$ und $R^5$ dieselbe Bedeutung wie in Formel I) unter 8 sowie $R^6$ und $R^7$ wie in Formel II) haben und $R^{3'}$ außerdem $OSi(CH_3)_3$, -OH, $CH_3CHOH$, $CH_2O$ Alkanoyl und $CH_3CHO$ Alkanoyl mit 1 bis 6 Kohlenstoffatomen sein kann, mittels einer Base in die entsprechenden Alkoholate überführt und diese anschließend durch Zugabe eines Alkylierungsmittels in die entsprechenden Etherumgewandelt (d.h. in eine Verbindung der allgemeinen Formel II, worin $R^8$ ein Alkylrest ist).
(Ausgangsprodukte : DD-PS 277685, DD-PS 251142)

Es werden dabei alle in der Verbindung der allgemeinen Formel IIB) vorhandenen Hydroxygruppen mitalkyliert.

Ethinylgruppen mit acidem Wasserstoff werden gegebenenfalls mitalkyliert.

Als Basen werden KOH, NaH, LiH, Lithiumalkyle wie Lithiumethyl, -n-butyl und tert.butyl, Calcium-, Lithium-, Natrium- oder Kaliumamide und Lithium-, Natrium- oder Kaliumnaphthalid oder durch elektrochemische Reduktion erzeugtes Naphthalidion eingesetzt, in einem Ether wie Diethylether, Tetrahydrofuran oder Dioxan, gegebenenfalls unter Zusatz eines Lösungsvermittlers wie Benzen oder Toluen, gearbeitet und als Alkylierungsmittel Dialkylsulfate wie Dimethyl- und Diethylsulfat, und Alkylhalogenide wie Methyl-, Ethyl-, Propyl-, Butyl-, Pentyl-, Hexyl-, Isopropylhalogenide in Form der Chloride, Bromide und Iodide, sowie Methylchloralkylether mit einem Alkylrest in der Bedeutung n = 1 - 6 eingesetzt.

Die Mitalkylierung gegebenenfalls vorhandener Ethinylgruppen ist bevorzugt.

Um O- und C-Alkylierung simultan vorzunehmen, ist sowohl die Basenmenge als auch die Menge an Alkylierungsmittel der Zahl der zu deprotonierenden Gruppen anzupassen.

Mit dem vorgeschlagenen Verfahren ist es möglich, ausgehend von 11β-Aryl-estr-9-en-5α-olen selektive Alkylierungen am Steroidmolekül vorzunehmen und damit einen Zugang zu biologisch aktiven Verbindungen zu - schaffen. Dabei wird der Umstand genutzt, daß sich 5α-OAlkylether, die selbst aufgrund ihrer strukturellen Merkmale von pharmakologischem Interesse sind, überraschenderweise leicht unter Abspaltung von Alkohol in die 4,9-Dien-3-one überführen lassen.

Hervorzuheben ist, daß die durch Alkylierung der 17-OH-Gruppe und gegebenenfalls der 17-Ethinylgruppe erhaltenen Produkte eine hohe antigestagene Wirksamkeit besitzen, wie Versuche an der Ratte zeigen. Die Ausgangsprodukte II, die für die Herstellung der Endverbindungen der allgemeinen Formel I mit den unter C) genannten Substituenten werden, wie in nachstehendem Schema angegeben ist, hergestellt.

Dabei werden

a) 17α-Alkoxymethyl-17β-hydroxy-estra-5(10), 9(11)-dien-3-ketale XII durch Deprotonierung mit Basen in 17β-Alkoholate XIII von 17α-Alkoxymethyl-estra-5(10), 9(11)-dien-3-ketalen überführt,
b) diese durch Alkylierung mit Alkylhalogeniden zu 17β-Alkoxy-17α-alkoxymethyl-estra-5(10),9(11)-dien-3-ketalen XIV umgesetzt,
c) diese selektiv mit $H_2O_2$ zu den 5α,10α-Epoxy-17β-alkoxy-17α-alkoxyethyl-estr-9(11)-en-3-ketalen XV epoxydiert und
d) diese mit Arylmagnesiumhalogeniden, die in der p-Stellung zum Magnesium eine Dimethylamino- oder eine geschützte Acetylgruppe besitzen, zu den 11β-arylsubstituierten 17β-Alkoxy-17α-alkoxymethyl-estr-9-en-5α-olen IIC) geöffnet.

Bevorzugt werden als 17α-Alkoxymethyl-17β-hydroxy-estra-5(10),9(11)-dien-ketale XII) die 3,3-Dimethylketale, als Base für die Deprotonierung Alkalinaphthalid und als organisches Lösungsmittel Tetrahydrofuran zur Bildung der Alkoholate der allgemeinen Formel XIII eingesetzt. Die Alkylierung wird mit Alkylhalogeniden, Alkyloxyalkylhalogeniden oder Alkylthioalkylhalogeniden direkt oder in Lösungsmitteln durchgeführt, wobei die 17β-Alkoxy-17α-alkoxymethyl-estra-5(10,9(11)-dien-3-ketale der allgemeinen Formel XIV entstehen. Diese werden durch die Epoxidation mit $H_2O_2$

und Halogenacetonen in Gegenwart von katalytischen Mengen tertiärer Basen oder mit Halogenaldehyden in Gegenwart von wasserbindenden Mitteln in inerten Lösungsmitteln in die 5α,10α-Epoxy-17β-alkoxy-17α-alkoxymethyl-est-9(11)-en-3-ketale der allgemeinen Formel XV überführt und diese durch Grignardierung in einem Ether in Gegenwart von Kupfer-I-salzen mit einem p-Brom-(2'-methyl-1',3'-dioxolan-2'-yl)-benzol zu den 11β-Aryl-substituierten 17β-Alkoxy-17α-alkoxymethyl-estr-9-en-5α-olen der allgemeinen Formel IIC) umgesetzt.

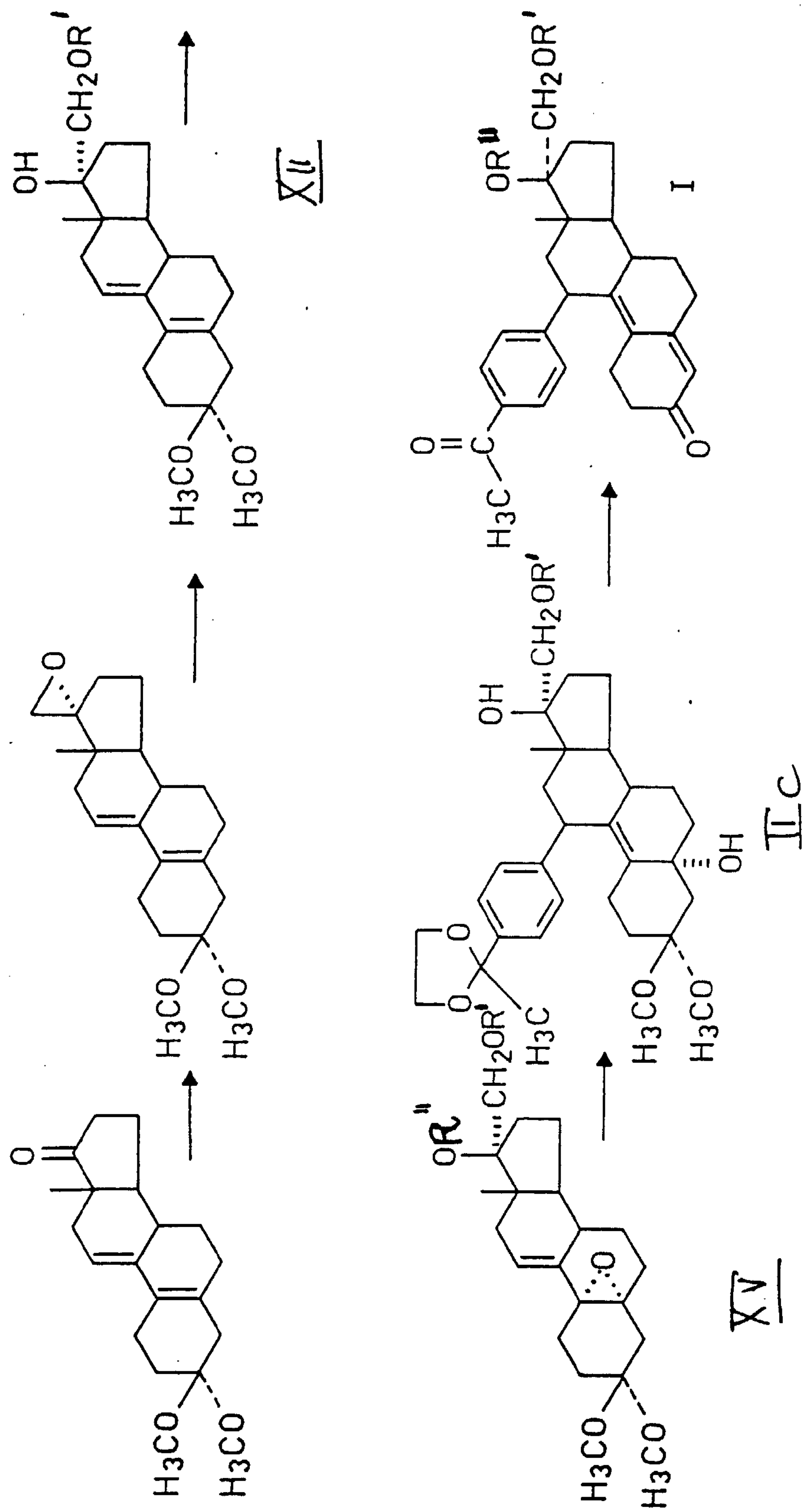

XII

I

IIc

XV

Insbesondere werden 17α-Alkoxymethyl-17β-hydroxy-estra-5(10),9(11)-dien-3-dimethylketale der allgemeinen Formel XII mit Natrium- oder Lithiumnaphthalid in Tetrahydrofuran deprotoniert und mit Alkylhalogeniden, Alkyloxyalkylhalogeniden, Alkylthioalkylhalogeniden direkt oder in Ether bei Temperaturen 20°C zu den

17β-Alkoxy-17α-alkoxymethyl-estra-5(10),9(11)-dien-3-ketalen der allgemeinen Formel XIV umgesetzt. Die Epoxidation mit $H_2O_2$ und Hexachloraceton oder Hexafluoaceton erfolgt in Gegenwart von katalytischen Mengen Triethylamin oder Pyridin, oder aber mit Chloralhydrat in Gegenwart von wasserfreiem primärem Alkaliphosphat und Alkalibicarbonat oder Alkalicarbonat bei Temperaturen um 20°C in Methylenchlorid oder Chloroform, wobei die 5α,10α-Epoxy-17β-alkoxy-17α-alkoxymethyl-estr-9(11)-en-3-ketale der allgemeinen Formel XV entstehen. Durch Grignardierung in Tetrahydrofuran mit CuCl und p-Brom(2'-methyl-1',3'-dioxolan-2'-yl')-benzol bei Temperaturen zwischen 0°C und 30°C werden die 11β-arylsubstituierten 17β-Alkoxy-17α-alkoxymethyl-estr-9-en-5a-olen der allgemeinen Formel II gebildet.

Die nachstehenden Beispiele dienen der näheren Erläuterung der Erfindung:

<u>Beispiel 1</u>

**a) 17α-Ethinyl-17β-hydroxy-13-methyl-gon-5(10)-en-3-on**

10 g 3,3-Dimethoxy-17α-ethinyl-13-methyl-gon-5(10)-en-17β-ol werden in 165 ml 80prozentigem wäßrigem Aceton suspendiert und unter kräftigem Rühren mit 0,2 ml 25prozentiger Schwefelsäure versetzt. Anschließend wird so lange bei Raumtemperatur gerührt, bis alles Ausgangsmaterial umgesetzt ist. Dann wird das Steroid durch Zugabe von Wasser ausgefällt, abgetrennt und getrocknet. Man erhält 9 g der 3-Keto-5(10)en-verbindung, die aus Aceton/Wasser umkristallisiert werden kann.

F.: Rohprodukt: 173°C bis 174°C

**b) 17α-Ethinyl-17β-hydroxy-13-methyl-gona-4,9-dien-3-on**

15 g 17α-Ethinyl-17β-hydroxy-13-methyl-gon-5(10)-en-3-on werden in 219 ml Pyridin gelöst und unter Kühlung (-5°C) mit 18,6 g Pyridinhydrobromidperbromid auf einmal versetzt. Nach 15 Minuten wird die Kühlung weggenommen und die sich langsam auf Raumtemperatur erwärmende Lösung noch ca. 15 Minuten gerührt und anschließend 2 ml Methylbuten zugegeben, um überschüssiges Bromierungsmittel zu zersetzen. Dann wird 5 Stunden bei Raumtemperatur gerührt und anschließend in Eiswasser, dem vorher etwas Salzsäure zugefügt wird, eingerührt. Nach dem Auskristallisieren wird das Steroid abgesaugt und gegebenenfalls aus Ether kristallisiert. Man erhält 14 g des Dienons (92,7% d. Th.).

F.: Rohprodukt: 168°C bis 173°C

**c) 17α-Ethinyl-3,3-ethylendioxy-13-methyl-gona-5(10),9(11)-dien-17β-ol**

2 g 17α-Ethinyl-17β-hydroxy-13-methyl-gona-4,9-dien-3-on werden in 3 ml Ethylenglykol, 5 ml Orthoameisensäuretriethylester und 30 ml Methylenchlorid gelöst und mit 0,1 g p-Toluolsulfonsäure versetzt. Man destilliert etwas Lösungsmittel ab und erhält eine dunkelbraune Lösung, die man 30 Minuten bei Raumtemperatur rührt. Danach wird mit einer wäßrigen Natriumbicarbonatlösung versetzt und das Steroid mit Methylenchlorid extrahiert. Nach dem Einengen der Extrakte wird der ölige Rückstand aus Ether/Hexan kristallisiert, wobei 2 g des Ketals erhalten werden.

F.: 152°C bis 155°C

IR[$cm^{-1}$]: kein C=O, 3300 (Ethinyl), 3600 (OH)

**c') 17α-Ethinyl-3,3-ethylendioxy-13-methyl-gona-5(10),9(11)-dien-17β-ol**

42,5g 17α-Ethinyl-17β-hydroxy-13-methyl-gona-4,9-dien-3-on (Rohprodukt) werden in 600 ml Benzol gelöst und mit 45 ml Ethylenglykol und 1,8 g p-Toluolsulfonsäure versetzt und so lange (ca. 2 Stunden) am Wasserabscheider unter intensiver Rührung gekocht, bis alles Ausgangsmaterial umgesetzt ist. Anschließend wird eine Natriumbicarbonatlösung zugefügt und das Steroid mit Benzol extrahiert. Der nach dem Einengen erhältliche ölige Rückstand wird aus Ether kristallisiert, wobei 43 g Ketal erhalten werden.

F.: 152°C bis 155°C

IR[$cm^{-1}$]: kein C=O, 3300 (Ethinyl), 3600 (OH)

**d) 3,3-Ethylendioxy-17α-ethinyl-17β-methoxy-13-methyl-gona-5(10),9(11)-dien**

7,5 g 3,3-Ethylendioxy-17α-ethinyl-13-methyl-gona-5(10),9(11)-dien-17β-ol (22,07 mmol Rohprodukt) werden in 25 ml Tetrahydrofuran gelöst und unter Inertgas mit 55 ml (22mmol, 0,4 m) einer etherischen Lithiummethyllösung in der Kälte (-10°C) versetzt. Bei der Zugabe setzt starke Gasentwicklung ein. Nach erfolgter Zugabe gibt man nach 15 Minuten 5,7 ml Methyliodid zu und erwärmt die Reaktionlösung auf Raumtemperatur. Man läßt über Nacht bei Raumtemperatur stehen, versetzt anschließend mit Wasser und extrahiert das Steroid mit Ether. Nach dem Einengen der

Extrakte wird an neutralem Aluminiumoxid chromatographiert. Als Elutionsmittel dient ein Benzol/Benzol-Essigester-Gemisch (5:1). Es werden 4,3 g des 17β-Methylethers in Form eines Öles isoliert, welcher direkt in die nächste Stufe eingesetzt werden kann.

IR[$cm^{-1}$]: kein C=O, 3300 (Ethinyl)

**d') 3,3-Ethylendioxy-17α-ethinyl-17β-methoxy-13-methyl-gona-5(10),9(11)-dien**

7 g 3,3-Ethylendioxy-17α-ethinyl-13-methyl-gona-5(10),9(11)-dien-17β-ol (20,6 mmol) werden in 50 ml Tetrahydrofuran gelöst und unter Stickstoff mit 1 g Naphthalin (7,8 mmol) und 0,2 g Lithium (28,57 mmol) versetzt und 5 Stunden bei ca. 70°C (Heizplattentemperatur) gerührt. Danach wurde unverbrauchtes Lithium entfernt und zu der Lösung bei Raumtemperatur 2,6 ml (41,72 mmol) Methyliodid hinzugefügt. Anschließend wird ca. 4 Stunden auf dem Wasserbad gelinde erwärmt und das Steriod nach Zugabe von Wasser mit Ether extrahiert. Das nach dem Einengen der Extrakte erhältliche Rohprodukt wird ohne weitere Reinigung in die nächste Stufe eingesetzt.

IR[$cm^{-1}$]: kein C=O, 3300 (Ethinyl)

**e) 17α-Ethinyl-3,3-ethylendioxy-17β-methoxy-5α,10α-epoxy-13-methyl-gon-9-en**

5 g 17α-Ethinyl-3,3-ethylendioxy-17β-methoxy-13-methyl-gona-5(10),9(11)-dien werden in 45 ml Methylenchlorid gelöst und nach Zugabe von 2 g $Na_2HPO_4$, 1 g $Na_2CO_3$ und 7,25 ml 30prozentigem $H_2O_2$ intensiv gerührt und anschließend mit 1,25 g (7,55 mmol) Chloralhydrat versetzt. Es wird ca. 20 Stunden bei Raumtemperatur gerührt und anschließend eine wäßrige Natriumcarbonatlösung zugegeben und das Steroid mit Methylenchlorid extrahiert. Die organische Phase wird wiederholt mit Natriumcarbonatlösung gewaschen, dann abgetrennt, getrocknet und eingeengt. Der verbleibende Rückstand wird an basischem Aluminiumoxid mit Benzol/Essigester (20:1) bis 10:1) flash chromatographiert. Das Epoxid wurde in Form eines Öles (2 g) isoliert und so in die nächste Stufe eingesetzt.

IR[$cm^{-1}$]: kein C=O

**f) 17α-Ethinyl-3,3-ethylendioxy-11β-[4-(2'-methyl-1',3'-dioxolan-2'-yl-) phenyl]-17β-methoxy-13-methyl-gon-9-en-5α-ol**

Ca. 1,5 g 17α-Ethinyl-3,3-ethylendioxy-17β-methoxy-5α,10α-epoxy-13-methyl-gon-9(11)-en (4,05 mmol) in 10 ml Tetrahydrofuran werden zu einer frisch bereiteten, auf -15°C abgekühlten Grignardlösung von 4-(2'-Methyl-1',3'-dioxolan-2'-yl)-phenylmagnesiumbromid (15 mmol) und 0,15 g CuCl in 30 ml Tetrahydrofuran zugetropft. Nach erfolgter Zugabe wird ca. 30 Minuten gerührt und dabei das Reaktionsgemisch allmählich auf Raumtemperatur erwärmt. Anschließend wird mit einer wäßrigen Ammoniumchloridlösung versetzt und das Steroid mit Ether extrahiert. Nach dem Einengen der Extrakte wird der verbleibende Rückstand an basischem Aluminiumoxid mit Benzol/Essigester (10:1) flash chromatographiert. Es werden 1,2 g der 11β-arylsubstituierten Verbindung isoliert, die aus Methanol oder Ethanol kristallisiert werden kann.

F.: 181°C bis 189°C $[\alpha]_D$: 31,1°

**g) 11β-(4-Acetylphenyl)-17α-ethinyl-17β-methoxy-13-methyl-gona-4,9-dien-3-on**

0,5 g 17α-Ethinyl-3,3-ethylendioxy-11β-[4-(2'methyl-1',3'-dioxolan-2'-yl-)penyl]-17β-methoxy-13-methyl-gon-9-en-5α-ol (0,94 mmol) werden mit 10 ml 70prozentiger wäßriger Essigsäure versetzt und 1 Stunde bei 70°C auf dem Wasserbad erwärmt. Dann wird das Steroid durch Zugabe von Wasser und etwas Ammoniak ausgefällt. Das isolierte Rohprodukt wird an neutralem Aluminiumoxid flash chromatographiert. Die Elution erfolgt mit Benzol/ Essigester-Gemisch (10:1). Man erhält 0,35 g der aus Methanol/Wasser in amorph kristalliner Form anfallenden 11β-Acetophenylverbindung.

F.: 87°C bis 91°C $[\alpha]_D$: 139,3°

<u>Beispiel 2</u>

Die Herstellung der Stufen a bis e erfolgt analog dem Beispiel 1.

**f) 11β-(4-Dimethylaminophenyl)-3,3-ethylendioxy-17α-propinyl-13-methyl-gon-9-en-5α,17β-diol**

Eine Grignardlösung in Tetrahydrofuran, bereitet aus 1,92 g Magnesium, 0,05 ml Dibromethan, 16,8 g p-Bromdimethylaminobenzol (80 mmol) und 120 ml Tetrahydrofuran, wird mittels Trockeneis auf ca. -15°C abgekühlt und mit 0,4 g CuCl versetzt. Nach 15minütigem Rühren werden in der Kälte ca. 9 g 3,3-Ethylendioxy-5α,10α-epoxy-17α-propinyl-13-methyl-gon-9(11)-en-17β-ol (Rohprodukt), welches in 15 ml Tetrahydrofuran gelöst ist, zugetropft. Anschließend rührt man 30 Minuten nach und läßt die Lösung langsam auf Raumtemperatur erwärmen. Zur Aufarbeitung wird mit wäßriger Ammoniumchloridlösung versetzt und das Steroid mit Ether extrahiert. Die organische Phase wird mit einer wäßrigen Natriumdisulfitlösung und zuletzt mit Wasser gewaschen, getrocknet und eingeengt. Der verbleibende Rückstand wird an basischem Aluminiumoxid chromatographiert. Die Elution erfolgt mit Benzol/Benzol-Essigester-Gemisch (10:1). Nach Umkristallisation aus Ether/n-Hexan werden 5,1 g der Dimethylaminophenylverbindung erhalten.

F.: 203°C bis 206°C

IR[$cm^{-1}$]: 3600 (OH), 3500 (OH - assoziiert)

**f') 11β-(4-Dimethylaminophenyl)-3,3-ethylendioxy-17β-methoxy-17α-propinyl-13-methyl-gon-9-en-5α-ol**

40 ml einer Grignardlösung (30 mmol) in Tetrahydrofuran, bereitet aus 0,96 g Magnesium, 0,05 ml Dibromethan, 8,4 g p-Brom-dimethylaminobenzol in 60 ml Tetrahydrofuran, werden mittels Trockeneis auf -15°C abgekühlt und mit 0,2 g CuCl versetzt. Nach 15minütigem Rühren unter Stickstoff werden in der Kälte 2,1 g 3,3-Ethylendioxy-17β-methoxy-5α,10α-epoxy-17α-propinyl-13-methyl-gon-9(11)-en, welches in 10 ml Tetrahydrofuran gelöst ist, zugetropft. Anschließend wird die externe Kühlung entfernt und 30 Minuten nachgerührt. Zur Aufarbeitung wird mit wäßriger Ammoniumchloridlösung versetzt und das Steroid mit Ether extrahiert. Dann wird mit wäßriger Natriumdisulfitlösung und zuletzt mit Wasser gewaschen, die organische Phase abgetrennt, getrocknet und eingeengt. Der verbleibende Rückstand wird an basischem Aluminiumoxid chromatographiert. Als mobile Phase dient Benzol und ein Benzol/Essigester-Gemisch (10:1). Nach Umkristallisation aus Ether/n-Hexan werden 1,5 g der 11β-Dimethylaminophenylverbindung erhalten.

F.: 124°C bis 127°C $[\alpha]_D$: -69,9°

**g) 11β-(4-Dimethylaminophenyl)-17β-hydroxy-17α-propinyl-13-methyl-gona-4,9-dien-3-on**

2,9 g 11β-(4-Dimethylaminophenyl)-3,3-ethylendioxy-17α-propinyl-13-methyl-gon-9-en-5α,17β-diol werden in 10 ml 70prozentiger wäßriger Essigsäure gelöst und eine Stunde auf dem Wasserbad (60°C bis 70°C) erwärmt. Anschließend wird das Steroid durch Zugabe von Wasser und 25prozentigem Ammoniak ausgefällt. Das anfallende Rohprodukt (ca. 2,5 g) wird an basischem Aluminiumoxid mit Benzol und Benzol/Essigester (10:1) chromatographiert. Nach Umkristallisation aus Acetonitril werden 1,3 g des Dienons erhalten.

F.: 178°C bis 181°C

**g') 11β-(4-Dimethylaminophenyl)-17β-methoxy-17α-propinyl-13-methyl-gona-4,9-dien-3-on**

0,7 g 11β-(4-Dimethylaminophenyl-3,3-ethylendioxy-17β-methoxy-17α-propinyl-13-methyl-gon-9-en-5α-ol werden in 10 ml 70prozentiger wäßriger Essigsäure gelöst und 1 Stunde auf dem Wasserbad (60°C bis 70°C) erwärmt. In Anschluß daran fällt man das Steroid durch Zugabe von Wasser und 25prozentigem Ammoniak aus. Das gut abfrittbare Rohprodukt wird an basischem Aluminiumoxid mit Benzol und Benzol/Essigester (10:1) flash chromatographiert. Der nach dem Einengen der Dienonfraktionen erhältliche Rückstand wird in Essigsäure gelöst und das Steroid durch Zugabe von Wasser und 25prozentigem Ammoniak ausgefällt. Man erhält 0,35 g des 11β-Dimethylaminophenyldienons.

F.: 101°C bis 105°C $[\alpha]_D$: 129,5°

<u>Beipiel 3</u>

Die Herstellung der Stufen a bis c erfolgt analog dem Beispiel 1.

**d) 3,3-Ethylendioxy-17β-methoxy-17α-propinyl-13-methyl-gona-5(10),9(11)-dien**

5 g 3,3-Ethylendioxy-17α-ethinyl-13-methyl-gona-5(10),9(11)-dien-17β-ol werden in 40 ml Tetrahydrofuran gelöst und unter Kühlung (-15°C) mit 48 ml Lithiumbutyl (0,61 m) langsam versetzt. Zu der homogenen Lösung gibt man 4,4 ml Methyliodid, die mit 4,4 ml Tetrahydrofuran verdünnt sind, langsam in der Kälte zu und erwärmt dann die Lösung unter Rühren auf Raumtemperatur. Nach 16 Stunden Reaktionszeit wird mit Wasser versetzt und das Steroid mit Ether extrahiert. Der nach dem Einengen der Etherextrakte erhältliche Rückstand kann ohne Reinigung in die nächste Stufe eingesetzt werden. Es werden 5 g der 17β-Methoxy-17α-propinylverbindung erhalten.

IR[$cm^{-1}$]: kein C=O, kein OH

MS: $M^+$ 368, ber. f. $C_{24}H_{32}O_3$

**e) 3,3-Ethylendioxy-17β-methoxy-5α,10α-epoxy-17α-propinyl-13-methyl-gon-9(11)-en**

5 g 3,3-Ethylendioxy-17β-methoxy-17α-propinyl-13-methyl-gona-5(10),9(11)-dien, 3 g wasserfreies $Na_2HPO_4$ und 2 g $Na_2CO_3$ werden in 25 ml Methylenchlorid suspendiert und unter Rühren bei Raumtemperatur mit 8 ml $H_2O_2$ (30prozentig) und zuletzt mit 1,5 g Chloralhydrat versetzt. Nach erfolgter Umsetzung wird eine wäßrige Natriumcarbonatlösung zugegeben und das Steroid mit Methylenchlorid extrahiert. Die organische Phase wird noch zweimal mit einer Natriumcarbonatlösung und zuletzt mit Wasser gewaschen, anschließend getrocknet und eingeengt. Das anfallende Rohprodukt wurde an basischem Aluminiumoxid mit Toluol/Toluol-Essigester-Gemisch (10:1) flash chromatographiert. Es wurden 2,1 g Epoxid in Form eines Öles isoliert, das direkt in die nächste Stufe eingesetzt wurde.

IR[$cm^{-1}$]: kein C=O

**f) 3,3-Ethylendioxy-17β-methoxy-11β-[4-(2'-methyl-1',3'-dioxolan-2'-yl-) phenyl]-17α-propinyl-13-methyl-gon-9-en-5α-ol**

Zu einer Suspension von 0,72 g Magnesiumspänen (30 mmol) in 5 ml Tetrahydrofuran fügt man 0,05 ml Dibromethan hinzu und setzt unter Argon sukzessive 55 ml einer 7,35 g p-Brom-(2'-methyl-1',3'-dioxolan-2'-yl-)benzol (30 mmol) enthaltenden Tetrahydrofuranlösung so zu, daß die Innentemperatur 45°C nicht übersteigt. In der Startphase wird leicht erwärmt (45°C) und danach die Temperatur durch Zugabe des Arylhalogenids geregelt. Nach erfolgter Zugabe des Arylhalogenids wird noch 2 Stunden bei 45°C nachgerührt. Von der so bereiteten Grignardlösung werden 30 ml entnommen und unter Kühlung (-5°C bis -15°C) mit 0,15 g CuCl versetzt. Man rührt 15 Minuten unter Beibehaltung dieser Temperatur und fügt dann in der Kälte eine Lösung von ca. 1 g 3,3-Ethylendioxy-17β-methoxy-5α,10α-epoxy-17α-propinyl-13-methyl-gon-9(11)-en in 10 ml Tetrahydrofuran hinzu. Anschließend wird 1 Stunde unter Feuchtigkeitsausschluß und Stickstoff gerührt und die Lösung allmählich auf Raumtemperatur erwärmt. Nach erfolgter Umsetzung wird mit wäßriger Ammoniumchloridlösung versetzt und das Steroid mit Ether extrahiert. Nach dem Einengen der Extrakte wird der verbleibende Rückstand an basischem Aluminiumoxid mit Benzol/Essigester (10:1) flash chromatographiert. Man erhält 1,05 g des Zielproduktes in Form eines amorphen Pulvers durch Fällung mittels Wasser aus der methanolischen Lösung.

IR[$cm^{-1}$]: 1600 (Aromat), 3500 (OH - assoziiert)

**g) 11β-(4-Acetylphenyl)-17β-methoxy-17α-propinyl-13-methyl-gona-4,9-dien-3-on**

0,15 g 3,3-Ethylendioxy-11β-[4-(2'-methyl-1',3'-dioxolan-2'-yl-)phenyl]-17β-methoxy-17α-propinyl-13-methyl-gon-9-en-5α-ol werden in 10 ml 70prozentiger wäßriger Essigsäure gelöst und auf dem Wasserbad (60°C bis 70°C) ca. 1 Stunde erwärmt. Danach wird die Lösung in der Kälte mit Wasser und etwas Ammoniak versetzt, wobei das Steroid in amorph kristalliner Form ausgefällt wird. Nach Chromatographie des Steroids an neutralem Aluminiumoxid mit Benzol/Essigester (10:1) werden 0,09 g des 4,9-Dien-3-ons isoliert, welches aus Acetonitril oder Ether kristallisiert werden kann.

F.: 110°C bis 113°C $[\alpha]_D$: 102,3°$_D$:

<u>Beispiel 4</u>

Die Herstellung der Stufen a bis c erfolgt analog dem Beispiel 1.

**d) 3,3-Ethylendioxy-17α-(3'-hydroxy-1'-propinyl)-17β-methoxy-13-methyl-gona-5(10),9(11)-dien**

4,3 g 3,3-Ethylendioxy-17α-ethinyl-17β-methoxy-13-methyl-gona-5(10),9(11)-dien werden in 10 ml Tetrahydrofuran gelöst und mit 37,5 ml frisch bereiteter Lithiummethyllösung (15 mmol), 0,4 m) versetzt. Anschließend gibt man bei Raumtemperatur 1 g Paraformaldehyd hinzu. Die Reaktion setzt sofort ein. Nach erfolgter Umsetzung wird Wasser hinzugegeben und das Steroid mit Ether extrahiert. Nach dem Einengen erhält man ca. 4,4 g Rohprodukt der hydroxymethylierten Verbindung, die ohne Reinigung in die nächste Stufe eingesetzt wird.

IR[$cm^{-1}$]: kein C=O, 3600 (OH)

**e) 3,3-Ethylendioxy-17α-(3'-hydroxy-1'-propinyll-17β-methoxy-5α,10α-oxido-13-methyl-gon-9(11)-en**

Ca. 4,4 g 3,3-Ethylendioxy-17α-(3'-hydroxy-1'-propinyl)-17β-methoxy-13-methyl-gona-5(10),9(11)-dien werden in 45 ml Methylenchlorid gelöst und nach Zugabe von 2 g $Na_2HPO_4$, 1 g $Na_2CO_3$ und 7,25 ml $H_2O_2$ (30prozentig) intensiv gerührt und anschließend mit 1,25 g Chloralhydrat (7,55 mmol) versetzt. Es wird 5 Stunden bei Raumtemperatur gerührt und 2,5 Tage im Kühlschrank aufbewahrt. Danach wird eine wäßrige Natriumcarbonatlösung zugegeben und das Steroid mit Methylenchlorid extrahiert. Die organische Phase wird wiederholt mit einer Natriumcarbonatlösung gewaschen, dann abgetrennt, getrocknet und eingeengt. Der verbleibende Rückstand wird an basischem Alumimium mit Benzol/Essigester (4:1 bis 2:1) flash chromatographiert. Es werden 3,5 g Epoxid in Form eines Öles isoliert, welches direkt in die nächste Stufe eingesetzt wird.

IR[$cm^{-1}$]: kein C=O, 3600 (OH)

**f) 3,3-Ethylendioxy-17α-(3'-hydroxy-1'-propinyl)-17β-methoxy-11β-[4-(2'-methyl-1',3'-dioxolan-2'-yl)-phenyl]-13-methyl-gon-9-en-5α-ol**

1 g 3,3-Ethylendioxy-17α-(3'-hydroxy-1'-propinyl)-17β-methoxy-5α,10α-epoxy-13-methyl-gon-9(11)-en (2,5 mmol) in 10 ml Tetrahydrofuran wird zu einer frisch bereiteten, auf -15°C abgekühlten Grignardlösung von 4-[2'-Methyl-1',3'-dioxolan-2'-yl)-phenylmagnesiumbromid (15 mmol) und 0,15 g CuCl in 30 ml Tetrahydrofuran zugetropft. Nach erfolgter Zugabe wird ca. 30 Minuten gerührt und das Reaktionsgemisch langsam auf Raumtemperatur erwärmt. Nach erfolgter Umsetzung wird mit einer wäßrigen Ammoniumchloridlösung versetzt und das Steroid mit Ether extrahiert. Nach dem Einengen der Extrakte wird der verbleibende Rückstand an basischem Aluminiumoxid flash chromatographiert. Als mobile Phase dient ein Benzol/Essigester-Gemisch (4:1 bis 1:1). Es wurden 0,5 g der 11β-arylsubstituierten Verbindung in Form eines Öles isoliert, welches direkt in die nächste Stufe eingesetzt wird.

IR[$cm^{-1}$]: 3600 (OH), 3500 (OH-assoziiert)

**g) 11β-(4-Acetylphenyl)-17α-(3'-hydroxy-1'(Z)-propenyl)-17β-methoxy-13-methyl-gona-4,9-dien-3-on**

Ca: 0,5 g 3,3-Ethylendioxy-17α-(3'-hydroxy-1'(Z)-propenyl)-17β-methoxy-11β-[4-(2-methyl-1',3'-dioxolan-2'-yl) phenyl]-13-methyl-gon-9-en-5α-ol (1,09 mmol) werden mit 10 ml 70prozentiger wäßriger Essigsäure versetzt und ca 1 Stunde bei 70°C auf dem Wasserbad erwärmt. Dann wird mit Wasser und etwas Ammoniak versetzt und das sich ölig abscheidende Produkt mit Methylenchlorid extrahiert. Der nach dem Einengen der Extrakte verbleibende Rückstand wird an neutralem Aluminiumoxid mit Benzol/Essigester (2:1) flash chromatographiert. Man erhält 0,25 g der aus Methanol kristallisierenden 11β-Acetophenylverbindung.

F.: 118°C bis 124°C $[\alpha]_D$: 188,2°

**f') 3,3-Ethylendioxy-17α-(3-hydroxy-1'(Z)-propenyl)-17β-methoxy-11β-[4-(2'-methyl-1',3'-dioxolan-2'-yl)-phenyl]-13-methyl-gon-9-en-5α-ol**

Ca. 0,5 g 3,3-Ethylendioxy-17α-[3'-hydroxy-1'-propinyl)-17β-methoxy-11β-[4-(2'-methyl-1',3'-dioxolan-2'-yl-)phenyl]-13-methyl-gon-9-en-5α-ol werden in 8 ml Tetrahydrofuran und 0,75 ml Pyridin gelöst und nach Zugabe von 0,1g $Pd/BaSO_4$ (10 %) bei Raumtemperatur in einer Wasserstoffatmosphäre unter Normaldruck bis zum Stillstand der Wasserstoffaufnahme hydriert. Nach wenigen Minuten ist der zunächst braun gefärbte Oxidkatalysator reduziert (schwarze Färbung). Nach einer Stunde ist die Wasserstoffaufnahme beendet. Es wird vom Katalysator abfiltriert und das zur Trockne eingeengte Filtrat direkt in die nächste Stufe eingesetzt.

IR[$cm^{-1}$]: 3600 (OH), 3500 (OH - assoziiert)

Beispiel 5

**11β-(4-Acetylphenyl)-17β-methoxy-17α-methoxymethyl-estra-4,9-dien-3-on**

a) 2,6 g 3,3-Dimethoxy-17α-methoxymethyl-estra-5(10),9(11)-dien-17β-ol werden in 20 ml THF gelöst und bei 20°C zu einer Lösung von Natriumnaphthalid (hergestellt aus 1,28 g Naphthalin und 250 mg Natrium in 50 ml THF) zugetropft, wobei das Alkoholat gebildet wird.

b) Zur dunkelgrünen Lösung fügt man nach 30 Minuten bei 20°C 5 ml Methyliodid und rührt 2 Stunden bei dieser Temperatur, zersetzt mit Methanol und anschließend mit Wasser, extrahiert mit Essigester, wäscht die organische Phase neutral, trocknet über $K_2CO_3$ und $Na_2SO_4$ und verdampft das Lösungsmittel unter Vakuum. Das Rohprodukt (6,7g) wird durch Säulenchromatographie an 120 g Aluminiumoxid (alkalisch) mit einem Benzol/Essigester-Gradienten gereinigt. Man erhält 2,36 g 17β-Methoxy-17α-methoxymethyl-estra-5(10),9(11)-dien-3,3-dimethylketal als farbloses Öl.

$[\alpha]_D$ = +110° ($CHCl_3$, C = 0,45)

c) In einer Mischung aus 15 ml Methylenchlorid und 0,5 ml Pyridin werden 1,88 g 17β-Methoxy-17α-methoxymethyl-estra-5(10),9(11)-dien-3,3-dimethylketal gelöst und bei 20°C mit 2 ml $H_2O_2$ und 0,2 ml Hexachloraceton versetzt. Nach 20 Stunden wird wäss. Bisulfit-Lösung zugegeben und mit Methylenchlorid extrahiert. Man wäscht neutral, trocknet über $K_2CO_3$ und $Na_2SO_4$ und destilliert das Lösungsmittel unter Vakuum ab. Das Rohprodukt wird durch Säulenchromatographie an 70g Aluminiumoxid (alkalisch) mit einem Benzol-Essigester-Gradienten gereinigt. Man erhält 1,24 g 5α,10α-Epoxy-17β-methoxy-17α-methoxymethyl-estr-9(11)-en-3,3-dimethyl-ketal.

Schmp. 116°C bis 119°C (Hexan)

$[\alpha]_D$ = + 7°C ($CHCl_3$)

d) Zu 486 mg Magnesium in 5 ml abs. Ether tropft man unter Argon 0,15 ml Dibromethan und anschließend 4,95 g 1-(4-Bromphenyl)-ethanon-1,1-ethylen-ketal in 30 ml abs. THF. Man rührt 3 Stunden bei 50°C Innentemperatur, kühlt auf 0°C ab und fügt 170 mg CuCl hinzu. Nach weiteren 20 Minuten werden 1,18 g 5α,10α-Epoxy-17β-methoxy-17α-methoxymethyl-estr-9(11)-en-3,3-dimethylketal in 20 ml THF zugetropft. Es wird 2 Stunden bei Raumtemperatur gerührt, der Ansatz mit wäss. Ammoniumchlorid-Lösung zersetzt und mit Essigester extrahiert. Die organische Phase wird neutral gewaschen über $Na_2SO_4$ getrocknet und unter Vakuum verdampft. Das Rohprodukt wird durch Säulenchromatographie an 70 g Aluminiumoxid (alkalisch) mit einem Benzol/Essigester-Gradienten gereinigt. Man erhält 970 mg 3,3-Dimethoxy-11β-[4-(1,1-ethylendioxyethyl)-phenyl]-17β-methoxy-17α-methoxymethyl-estr-9-en-5α-ol als farbloses Öl, das direkt weiterverarbeitet wird.

e) 970 mg 3,3-Dimethoxy-11β-[4-(1,1-ethylendioxyethyl)-phenyl]-17β-methoxy-17α-methoxymethyl-estr-9-en-5α-ol werden in 20 ml verdünnter Essigsäure 20 Stunden bei Raumtemperatur gerührt. Nach Zugabe von Wasser wird mit Essigester extrahiert, die organische Phase mit verdünnter wäss. NaOH und Wasser gewaschen, über $Na_2SO_4$ getrocknet und unter Vakuum verdampft. Nach Umkristallisation aus Ether werden 430 mg 11β-(4-Acetylphenyl)-17β-methoxy-17α-methoxymethyl-estra-4,9-dien-3-on erhalten.

Schmp. 133°C bis 137°C (Ether)

$[\alpha]_D$ = + 170°C ($CHCl_3$)

Beispiel 6

**11β-(4-Acetylphenyl)-17α-ethoxymethyl-17β-methoxy-estra-4,9-dien-3-on**

a) Zu 1,88 g 3,3-Dimethoxy-17α-ethoxymethyl-estra-5(10),9(11)-dien-17β-ol in 30 ml THF tropft man solange eine Natriumnaphthalid-Lösung (hergestellt aus 250 mg Natrium und 1,28 g Naphthalin in 50 ml THF) bis eine dunkelgrüne Färbung bestehen bleibt, wobei das Alkoholat gebildet wird.

b) Nach 30 Minuten bei Raumtemperatur fügt man 4 ml Methyliodid zu und rührt weitere 2 Stunden nach. Der Ansatz wird mit wäss. $NH_4Cl$-Lösung zersetzt, mit Essigester extrahiert. Man wäscht die organische Phase neutral, trocknet über $K_2CO_3$ und $Na_2SO_4$ und verdampft das Lösungsmittel unter Vakuum. Das Rohprodukt wird durch Säulenchromatographie an Aluminiumoxid (alkalisch) mit einem Benzol/Essigester-Gradienten gereinigt. Man erhält 1,56 g 17α-Ethoxymethyl-17β-methoxy-estra-5(10),9(11)-dien-3,3-demethylketal als farbloses Öl, das direkt in die nächste Stufe eingesetzt wird.

c) 1,56 g 17α-Ethoxymethyl-17β-methoxy-estra-5(10),9(11)-dien-3,3-dimethyl-ketal werden in 20 ml Methylenchlorid und 0,5 ml Pyridin gelöst und bei 20°C mit 3 ml $H_2O_2$ und 0,3 ml Hexachloraceton versetzt. Nach 20 Stunden wird wäss. Bisulfit-Lösung zugegeben und mit Methylenchlorid extrahiert. Man wäscht neutral, trocknet über $K_2CO_3$ und $Na_2SO_4$ und destilliert das Lösungsmittel unter Vakuum ab. Das Rohprodukt wird durch Säulenchromatographie an 60 g Aluminiumoxid (alkalisch) mit einem Benzol/Essigester-Gradienten gereinigt. Man erhält 940 mg 5α, 10α-Epoxy-17α-ethoxymethyl-17β-methoxy-estr-9(11)-en-3,3-dimethylketal als farbloses Öl, das direkt in die nächste Stufe eingesetzt wird.

d) Zu 486 mg Magnesium in 5 ml abs. Ether tropft man unter Argon 0,15 ml Dibromethan und anschließend 4,95 g 1-(4-Bromphenyl)-ethanon-1,1-ethylen-ketal in 30 ml abs. THF. Man rührt 3 Stunden bei 50°C Innentemperatur, kühlt auf 0°C ab und fügt 170 mg CuCl hinzu. Nach weiteren 20 Minuten werden 940 mg 5α,10α-Epoxy-17α-ethoxymethyl-17β-methoxy-estr-9-(11)-en-3,3-dimethylketal in 20 ml THF zugetropft. Es wird 2 Stunden bei Raumtemperatur gerührt, der Ansatz mit wäss. Ammoniumchlorid-Lösung zersetzt und mit Essigester extrahiert. Die organische Phase wird neutral gewaschen, über $Na_2SO_4$ getrocknet und unter Vakuum verdampft. Das Rohprodukt wird durch Säulenchromatographie an 60 g Aluminiumoxid (alkalisch) mit einem Benzol/Essigester-Gradienten gereinigt. Man erhalt 700 mg 3,3-Dimethoxy-11β-[4-(1,1-ethylendioxyethyl)-phenyl]-17α-ethoxymethyl-17β-methoxy-estr-9-en-5α-ol als farbloses Öl, das direkt weiterverarbeitet wird.

e) 700 mg 3,3-Dimethoxy-11β-[4-(1,1-ethylendioxyethyl)-phenyl]-17α-ethoxy-methyl-17β-methoxy-estr-9-en-5α-ol werden in 20 ml verdünnter Essigsäure (70 %ig) 20 Stunden bei Raumtemperatur gerührt. Nach Zugabe von Wasser wird mit Essigester extrahiert, die organische Phase mit verdünnter wäss. NaOH und Wasser gewaschen, über $Na_2SO_4$ getrocknet und unter Vakuum verdampft. Das Rohprodukt wird durch präparative Schichtchromatographie an Kieselgel 60 mit dem Laufmittelgemisch Benzol/Aceton 9:1 (v/v) gereinigt. Es werden 290 mg 11β-(4-Acetylphenyl)-17α-ethoxymethyl-17β-methoxy-estra-4,9-dien-3-on erhalten.

Schmp. 134°C bis 137°C (Aceton/Ether)

$[\alpha]_D = + 180°$ ($CHCl_3$)

Beispiel 7

**11β-(4-Acetylphenyl)-17β-methoxy-17α-propoxymethyl-estra-4,9-dien-3-on**

a) Zu 3,9 g 3,3-Dimethoxy-17α-propoxymethyl-estra-5(10),9(11)-dien-17β-ol in 30 ml THF tropft man solange eine Natriumnaphthalid-Lösung (hergestellt aus 375 mg Natrium und 1,9 g Naphthalin in 100 ml THF) bis eine dunkelgrüne Färbung bestehen bleibt, wobei das Alkoholat gebildet wird.

b) Nach 30 Minuten Rührung bei 20°C tropft man zur Lösung 8 ml Methyliodid langsam hinzu und rührt weitere 3 Stunden bei Raumtemperatur. Nach Zugabe von wäss. $NH_4Cl$-Lösung wird mehrmals mit Essigester extrahiert. Man wäscht die organische Phase neutral, trocknet über $K_2CO_3$ und $Na_2SO_4$ und verdampft das Lösungsmittel unter Vakuum. Das Rohprodukt wird durch Säulenchromatographie an 140 g Aluminiumoxid (alkalisch) mit einem Benzol/ Essigester-Gradienten gereinigt. Man erhält 3,56 g 17β-Methoxy-17α-propoxymethyl-estra-5(10),9(11)-dien-3,3-dimethylketal als farbloses Öl, das direkt in die nächste Stufe eingesetzt wird.

c) Zu 3,56 g 17β-Methoxy-17α-propoxymethyl-estra-5(10),9(11)-dien-3,3-dimethylketal in 20 ml Methylenchlorid werden 1 g $NaHCO_3$, 2,5 g $NaH_2PO_4$, 4,5 ml $H_2O_2$ und 1,5 g Chloralhydrat gegeben. Man rührt ca. 3,5 Stunden bis zum vollständigen Umsatz bei Raumtemperatur, gibt Wasser zu und extrahiert mit Methylenchlorid. Die organische Phase wird nacheinander mit wäss. Bisulfit-Lösung, $K_2CO_3$-Lösung und Wasser gewaschen, über $Na_2SO_4$ getrocknet und unter Vakuum verdampft. Das Rohprodukt wird durch Säulenchromatographie an 120 g Aluminiumoxid (alkalisch) mit einem Benzol/Essigester-Gradienten gereinigt. Man erhält 2,45 g 5α,10α-Epoxy-17β-methoxy-17α-propoxymethyl-estr-9(11)-en-3,3-dimethylketal als farbloses Öl,das direkt in die nächste Stufe eingesetzt wird.

d) Zu 560 mg Magnesium in 5 ml abs. Ether gibt man nacheinander 0,2 ml Dibromethan und 5,7 g 1-(4-Bromphenyl)-ethanon-1,1-ethylenketal in 30 ml abs. THF. Man rührt 3 Stunden bei 50°C Innentemperatur, kühlt auf 0°C ab und fügt 220 mg CuCl hinzu. Nach weiteren 20 Minuten werden 2,45 g 5α,10α-Epoxy-17β-methoxy-17α-propoxymethyl-estr-9(11)-en-3,3-dimethylketal in 40 ml abs. THF zugetropft. Man rührt 3 Stunden bei Raumtemperatur, zersetzt mit wäss. $NH_4Cl$-Lösung und extrahiert mit Essigester. Es wird farblos und neutral gewaschen, über $Mg_2SO_4$ getrocknet und im Vakuum eingedampft. Das Rohprodukt wird an 100 g Aluminiumoxid (alkalisch) mit einem Benzol/Essigester-Gradienten gereinigt. Man erhält 1,5 g 3,3-Dimethoxy-11β-[4-(1,1-ethylendioxyethyl)-phenyl]-17β-methoxy-17α-propoxymethyl-estr-9-en-5α-ol als farbloses Öl, das direkt in die nächste Stufe eingesetzt wird.

e) Zu 1,5 g 3,3-Dimethoxy-11β-[4-(1,1-ethylendioxyethyl)-phenyl]-17β-methoxy-17α-propoxymethyl-estr-9-en-5α-ol werden 20 ml verdünnter Essigsäure (70 %ig) gegeben und die Mischung 4 Stunden bei Raumtemperatur gerührt. Nach Zugabe von Wasser wird mit Essigester extrahiert, die organische Phase mit verdünnter wäss. NaOH und Wasser gewaschen, über $Na_2SO_4$ getrocknet und unter Vakuum verdampft. Das Rohprodukt wird aus Ether/ Aceton/Hexan umkristallisiert. Man erhält 790 mg 11β-(4-Acetylphenyl)-17β-methoxy-17α-propoxymethyl-estra-4,9-dien-3-on .

Schmp. 118°C bis 120°C

$[\alpha]_D = + 160°$ ($CHCl_3$)

Beispiel 8

Stufen a) bis c) wie unter Beispiel 5.

d) Zu 850 mg Magnesium in 10 ml abs. THF tropft man unter Argon 0,15 ml Dibromethan und anschließend 5 g p-Bromdimethylanilin in 15 ml abs. THF. Man rührt 3 Stunden bei 50°C Innentemperatur, kühlt auf -20°C ab und

fügt 200 mg CuCl hinzu. Nach weiteren 20 Minuten werden 1,72 g 5α,10α-Epoxy-17β-methoxy-17α-methoxymethyl-estr-9(11)-en-3,3-dimethylketal in 15 ml THF zugetropft. Es wird 1 Stunde bei 10°C bis 15°C und dann bei Raumtemperatur gerührt, der Ansatz mit wäss. Ammoniumchlorid-Lösung zersetzt und mit Ether extrahiert. Die organische Phase wird neutral gewaschen, über $Na_2SO_4$ getrocknet und unter Vakuum verdampft. Das Rohprodukt wird durch Säulenchromatographie an 70g Aluminiumoxid (alkalisch) mit einem Benzol/Essigester-Gradienten gereinigt. Man erhält 1,1 g 3,3-Dimethoxy-11β-(4-dimethylaminophenyl)-17β-methoxy-17α-methoxymethyl-estr-9-en-5α-ol als farbloses Öl, das direkt weiterverarbeitet wird.

e) 700 mg 3,3-Dimethoxy-11β-(4-dimethylaminophenyl)-17β-methoxy-17α-methoxymethyl-estr-9-en-5α-ol werden in 20 ml verdünnter Essigsäure 20 Stunden bei Raumtemperatur gerührt. Nach Zugabe von Wasser wird mit Essigester extrahiert, die organische Phase mit verdünnter wäss. NaOH und Wasser gewaschen, über $Na_2SO_4$ getrocknet und unter Vakuum verdampft. Nach mehrmaliger präparativer Schichtchromatographie an Aluminiumoxid (neutral) und Kieselgel $PF_{254}$ werden 420 mg 11β-(4-Dimethylaminophenyl)-17β-methoxy-17α-methoxymethyl-estra-4,9-dien-3-on als Schaum erhalten.

$[\alpha]_D = +167°$ ($CHCl_3$)

$^1$H-NMR ($CDCl_3$, TMS):

δ (ppm): 0,59 (s, 3H, H-18); 2,95 (s, 6H, $N(CH_3)_2$ ; 3,26 (s, 3H, 17β-$OCH_3$) 3,41 (s, 3H, 17α-$CH_2OC\underline{H}_3$); 3,30 - 3,64 (m, 2H, ABX-System, 17α.-$C\underline{H}_2OCH_3$); 4,30 (d, J = 7 Hz, 1H, H-11); 5,75 (s, 1H, H-4); 6,60 - 7,06 (m, 4H, AA'BB'-System des Aromaten).

## Patentansprüche

### Patentansprüche für folgende Vertragsstaaten : AT, BE, CH, DE, DK, FR, GB, IT, LI, LU, NL, SE

**1.** 11β-Aryl-gona-4,9-diene der allgemeinen Formel I

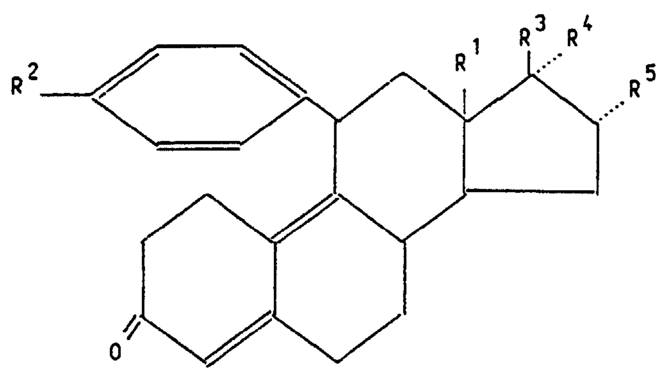

(I)

worin

A)

$R^1$ eine Methyl- oder Ethylgruppe,

$R^2$ eine Alkoxy-, Alkylthio-, wobei unter Alkyl ein Alkyl-, Alkenyl- oder eine entsprechende cyclische Verbindung mit 1 bzw. 2 bzw. 3 bis 7 Kohlenstoffatomen zu verstehen ist, eine Dimethylamino-, Monomethylamino-, Cyano-, Formyl-, Acetyl-, oder 1-Hydroxyethylgruppe,

$R^3$ eine Alkoxymethoxy- oder Alkoxygruppe mit jeweils 1 bis 6 Kohlenstoffatomen, und

$R^4$ eine Ethinyl-, Prop-1-inyl-, 3-Hydroxyprop-1-inyl-, 3-Alkanoyloxyprop-1-inyl-, 3-Alkanoyloxyprop-1-enyl-, 3-Alkanoyloxypropylgruppe mit jeweils 1 bis 6 Kohlenstoffatomen, 3-Hydroxyprop-1-enyl- und 3-Hydroxypropylgruppe sowie

$R^5$ ein Wasserstoffatom

oder

B)

$R^1$ eine Methyl- oder Ethylgruppe,
$R^2$ eine Methoxy-, Thiomethyl-, Dimethylamino-, Monomethylamino-, Cyano-, Formyl-, Acetyl- oder 1-Alkoxyethylgruppe mit 1 bis 6 Kohlenstoffatomen im Alkoxyrest,
$R^3$ eine Alkoxy-, Alkoxymethyloxy-, Alkanoyloxyethyloxy, mit jeweils 1 bis 6 Kohlenstoffatomen im Alkoxyrest, und
$R^4$ eine Ethinyl-, Prop-1-inyl-, Alkyl-, 3-Alkoxyprop-1-inyl-, 3-Alkoxypropylgruppe mit jeweils 1 bis Kohlenstoffatomen im Alkyl- bzw. Alkoxyrest oder die Gruppierung $-CH_2Y$, worin Y ein Cyano-, Azido- oder Alkoxyrest mit 1 bis 6 Kohlenstoffatomen sowie
$R^5$ ein Wasserstoffatom oder eine Alkylgruppe mit 1 bis 4 Kohlenstoffatomen oder aber
$R^4$ und $R^5$ gemeinsam eine Tetramethylenbrücke

oder
C)

$R^1$ eine Methylgruppe,
$R^2$ eine Dimethylamino, eine freie oder ketalisierte Acetylgruppe,
$R^3$ eine Alkoxygruppe mit 1 bis 6 Kohlenstoffatomen oder Alkylthiomethyloxygruppe mit 1 bis 4 Kohlenstoffatomen im Alkylrest,
$R^4$ eine Alkoxymethylgruppe mit 1 bis 6 Kohlenstoffatomen im Alkoxyrest sowie
$R^5$ ein Wasserstoffatom

bedeutet.

**2.** Verbindungen der allgemeinen Formel I,
worin

$R^1$ eine Hethylgruppe,
$R^2$ eine Dimethylamino- oder Acetylgruppe und außerdem

in A)

$R^3$ eine Methoxygruppe.
$R^4$ eine Prop-1-inyl-, Ethinyl-, 3-Hydroxy-prop-1-inyl-, 3-Hydroxy-prop-1(Z)-enyl-, 3-Hydroxypropylgruppe und
$R^5$ ein Wasserstoffatom,

in B)

$R^3$ eine Methoxygruppe,
$R^4$ eine Prop-1-inyl-, Ethinyl- oder 3-Hydroxypropylgruppe,
$R^5$ ein Wasserstoffatom oder
$R^4$ und $R^5$gemeinsam eine Tetramethylenbrücke,

in C)

$R^3$ eine Methoxygruppe,
$R^4$ eine Methoxymethyl-, Ethoxymethyl- oder Propoxymethylgruppe und
$R^5$ ein Wasserstoff

bedeutet.

**3.** 11β-(4-Dimethylaminophenyl)-17β-methoxy-17α-propinyl-13-methyl-gona-4,9-dien-3-on,
11β-(4-Acetylphenyl)-17β-methoxy-17α-propinyl-13-methyl-gona-4,9-dien-3-on,
11β-(4-Dimethylaminophenyl)-17β-methoxy-17α-ethinyl-13-methyl-gona-4,9-dien-3-on,
11β-(4-Acetylphenyl)-17β-methoxy-17α-ethinyl-13-methyl-gona-4,9-dien-3-on,
11β-(4-Acetylphenyl)-17α-(3'-hydroxy-1-propinyl)-17β-methoxy-13-methyl-gona-4,9-dien-3-on,
11β-(4-Acetylphenyl)-17α-(3'-hydroxy-1'(Z)-propenyl)-17β-methoxy-13-methyl-gona-4,9-dien-3-on sowie
11β-(4-Acetylphenyl)-17α-(3'-hydroxypropyl)-17β-methoxy-13-methyl-gona-4,9-dien-3-on,
11β-(4-Acetylphenyl)-17β-methoxy-17α-methoxymethyl-estra-4,9-dien-3-on,

11β-(4-Dimethylaminophenyl)-17β-methoxy-17α-methoxymethyl-estra-4,9-dien-3-on,
11β-(4-Acetylphenyl)-17α-ethoxymethyl-17β-methoxy-estra-4,9-dien-3-on,
11β-(4-Acetylphenyl)-17β-methoxy-17α-propoxymethyl-estra-4,9-dien-3-on,
11β-(4-Dimethylaminophenyl)-17β-methoxy-17α-propinyl-13-methyl-gona-4,9-dien-3-on,
11β-(4-Methoxyphenyl)-17β-methoxy-17α-propinyl-13-methyl-gona-4,9-dien-3-on,
11β-(4-Dimethylaminophenyl)-17α-(3'-hydroxy-1'-Z-propenyl)-17β-methoxy-13-methyl-gona-4,9-dien-3-on,
11β-(4-Methoxyphenyl)-17β-methoxy-17α-(3'-hydroxy-1'-Z-propenyl)-13-methyl-gona-4,9-dien-3-on,
11β-(4-Dimethylaminophenyl)-17α-(3'-hydroxypropyl)-17β-methoxy-13-methyl-gona-4,9-dien-3-on
11β-(4-Methoxyphenyl)-17α-(3'-hydroxypropyl)-17β-methoxy-13-methyl-gona-4,9-dien-3-on
11β-(4-Dimethylaminophenyl)-17α-(3'-hydroxy-1'-propinyl)-17β-methoxy-13-methyl-gona-4,9-dien-3-on,
11β-(4-Methoxyphenyl)-17α-(3'-hydroxy-1'-propinyl)-17β-methoxy-13-methyl-gona-4,9-dien-3-on,
11β-(4-Dimethylaminophenyl)-17α-ethinyl-17β-methoxy-13-methyl-gona-4,9-dien-3-on,
11β-(4-Methoxyphenyl)-17α-ethinyl-17β-methoxy-13-methyl-gona-4,9-dien-3-on,
11β-(4-Methoxyphenyl)-17β-methoxy-17α-methoxymethyl-13-methyl-gona-4,9-dien-3-on.

**4.** Verfahren zur Herstellung von 11β-Aryl-gona-4,9-dienen der allgemeinen Formel I

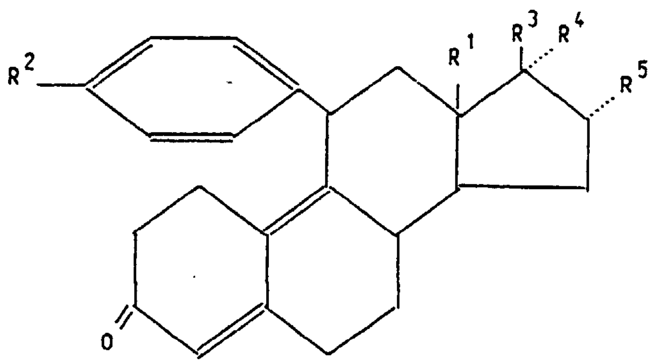

(I)

worin
$R^1$, $R^2$, $R^3$, $R^4$ sowie $R^5$ die in Anspruch 1 angegebene Bedeutung haben, dadurch gekennzeichnet, daß eine Verbindung der allgemeinen Formel II

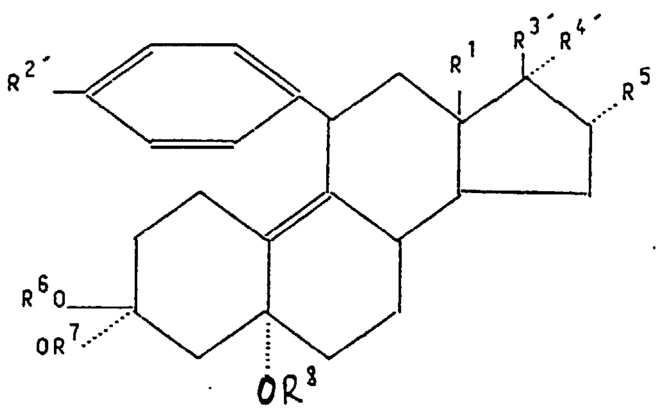

(II),

worin

$R^1$ und $R^5$ die in Formel I angegebene Bedeutung haben,
$R^{2'}$, $R^{3'}$ und $R^{4'}$ dieselbe Bedeutung wie $R^2$, $R^3$ und $R^4$ in Formel I haben, wobei gegebenenfalls vorhandene Keto- und/oder Hydroxygruppen geschützt sein können,
$R^6$ und $R^7$ je eine Methyl- oder Ethylgruppe oder gemeinsam eine Ethylen-oder 2,2-Dialkylpropylengruppe, insbesondere eine 2,2-Dimethylpropylengruppe sowie
$R^8$ ein Wasserstoffatom oder einen Alkylrest mit 1 bis 6 Kohlenstoffatomen

bedeuten,
durch Säurebehandlung in einem mit Wasser mischbaren Lösungsmittel, gegebenenfalls unter Erwärmen auf 60°-80°C, in eine Verbindung der allgemeinen Formel I überführt wird.

**5.** Verfahren nach Anspruch 4, dadurch gekennzeichnet, daß die Säurebehandlung mit Essigsäure, p-Toluolsulfonsäure oder mit Mineralsäuren wie Salzsäure, Schwefelsäure, Phosphorsäure oder Perchlorsäure in Methanol, Ethanol oder Aceton durchgeführt wird.

**6.** Pharmazeutische Präparate, dadurch gekennzeichnet, daß sie mindestens eine Verbindung der allgemeinen Formel I sowie einen pharmazeutisch verträglichen Träger enthalten.

**7.** Verwendung von Verbindungen der allgemeinen Formel I zur Herstellung von Arzneimitteln.

**8.** Zwischenverbindungen der allgemeinen Formel II

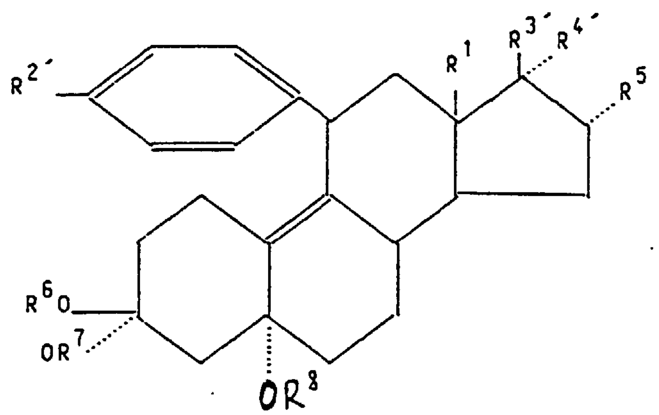

(II),

worin

$R^1$ und $R^5$ die in Formel I angegebene Bedeutung haben,
$R^{2'}$, $R^{3'}$ und $R^{4'}$ dieselbe Bedeutung wie $R^2$, $R^3$ und $R^4$ in Formel I haben, wobei gegebenenfalls vorhandene Keto- und/oder Hydroxygruppen geschützt sein können,
$R^6$ und $R^7$ je eine Methyl- oder Ethylgruppe oder gemeinsam eine Ethylen-oder 2,2-Dialkylpropylengruppe, insbesondere eine 2,2-Dimethylpropylengruppe sowie
$R^8$ ein Wasserstoffatom oder einen Alkylrest mit 1 bis 6 Kohlenstoffatomen

bedeuten.

**Patentansprüche für folgende Vertragsstaaten : ES, GR**

**1.** Verfahren zur Herstellung von 11β-Aryl-gona-4,9-dienen der allgemeinen Formel I

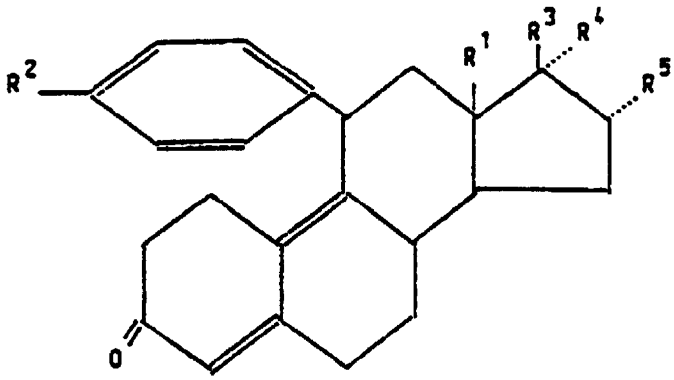

(I)

worin

A)

$R^1$ eine Methyl- oder Ethylgruppe,
$R^2$ eine Alkoxy-, Alkylthio-, wobei unter Alkyl ein Alkyl-, Alkenyl- oder eine entsprechende cyclische Verbindung mit 1 bzw. 2 bzw. 3 bis 7 Kohlenstoffatomen zu verstehen ist, eine Dimethylamino-, Monomethylamino-, Cyano-, Formyl-, Acetyl-, oder 1-Hydroxyethylgruppe,
$R^3$ eine Alkoxymethoxy-, oder Alkoxygruppe mit jeweils 1 bis 6 Kohlenstoffatomen, und
$R^4$ eine Ethinyl-, Prop-1-inyl-, 3-Hydroxyprop-1-inyl-, 3-Alkanoyloxyprop-1-inyl-, 3-Alkanoyloxyprop-1-enyl-, 3-Alkanoyloxypropylgruppe mit jeweils 1 bis 6 Kohlenstoffatomen, 3-Hydroxyprop-1-enyl- und 3-Hydroxypropylgruppe sowie
$R^5$ ein Wasserstoffatom

oder
B)

$R^1$ eine Methyl- oder Ethylgruppe,
$R^2$ eine Methoxy-, Thiomethyl-, Dimethylamino-, Monomethylamino-, Cyano-, Formyl-, Acetyl- odere 1-Alkoxyethylgruppe mit 1 bis 6 Kohlenstoffatomen im Alkoxyrest,
$R^3$ eine Alkoxy-, Alkoxymethyloxy-, Alkanoyloxyethyloxy-, mit jeweils 1 bis 6 Kohlenstoffatomen im Alkoxyrest, und
$R^4$ eine Ethinyl-, Prop-1-inyl-, Alkyl-, 3-Alkoxyprop-1-inyl-, 3-Alkoxypropylgruppe mit jeweils 1 bis 4 Kohlenstoffatomen im Alkyl- bzw. Alkoxyrest oder die Gruppierung $-CH_2Y$, worin Y ein Cyano-, Azido- oder Alkoxyrest mit 1 bis 6 Kohlenstoffatomen sowie
$R^5$ ein Wasserstoffatom oder eine Alkylgruppe mit 1 bis 4 Kohlenstoffatomen oder aber
$R^4$ und $R^5$ gemeinsam eine Tetramethylenbrücke

oder
C)

$R^1$ eine Methylgruppe,
$R^2$ eine Dimethylamino, eine freie oder ketalisierte Acetylgruppe,
$R^3$ eine Alkoxygruppe mit 1 bis 6 Kohlenstoffatomen oder Alkylthiomethyloxygruppe mit 1 bis 4 Kohlenstoffatomen im Alkylrest.
$R^4$ eine Alkoxymethylgruppe mit 1 bis 6 Kohlenstoffatomen im Alkoxyrest sowie
$R^5$ ein Wasserstoffatom

bedeutet.

dadurch gekennzeichnet, daß eine Verbindung der allgemeinen Formel II

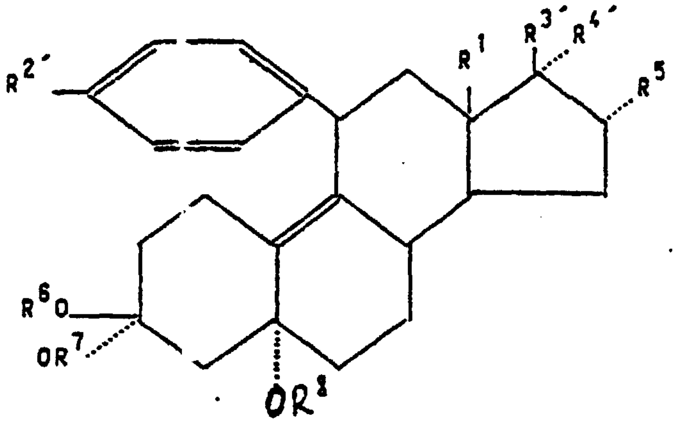

(II),

worin

$R^1$ und $R^5$ die in Formel I angegebene Bedeutung haben,
$R^{2'}$, $R^{3'}$ und $R^{4'}$ dieselbe Bedeutung wie $R^2$, $R^3$ und $R^4$ in Formel I haben, wobei gegebenenfalls vorhandene Keto- und/oder Hydroxygruppen geschützt sein können,
$R^6$ und $R^7$ je eine Methyl- oder Ethylgruppe oder gemeinsam eine Ethylen-oder 2,2-Dialkylpropylengruppe, insbesondere eine 2,2-Dimethylpropylengruppe sowie
$R^8$ ein Wasserstoffatom oder einen Alkylrest mit 1 bis 6 Kohlenstoffatomen

bedeuten,
durch Säurebehandlung in einem mit Wasser mischbaren Lösungsmittel, gegebenenfalls unter Erwärmen auf 60°-80°C, in eine Verbindung der allgemeinen Formel I überführt wird.

## Claims

**Claims for the following Contracting States : AT, BE, CH, DE, DK, FR, GB, IT, LI, LU, NL, SE**

**1.** 11β-Aryl-gona-4,9-dienes of the general formula I

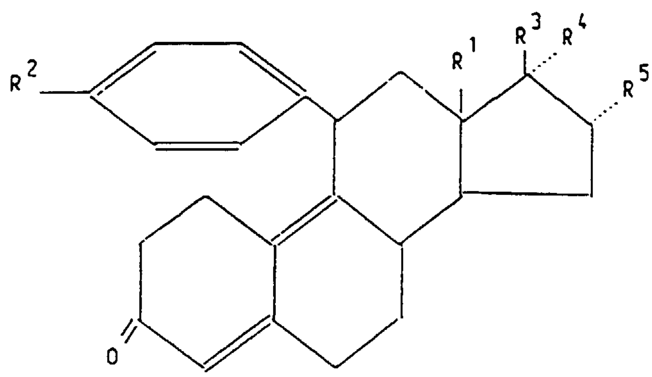

(I)

wherein

A)

$R^1$ is a methyl or ethyl group,
$R^2$ is an alkoxy or alkylthio group, wherein alkyl is to be understood as being alkyl, alkenyl or a corresponding cyclic compound having 1 or 2 or 3 to 7 carbon atoms, respectively, or a dimethylamino, monomethylamino, cyano, formyl, acetyl or 1-hydroxyethyl group,

$R^3$ is an alkoxymethoxy or alkoxy group each having from 1 to 6 carbon atoms, and
$R^4$ is an ethynyl, prop-1-ynyl, 3-hydroxyprop-1-ynyl, 3-alkanoyloxyprop-1-ynyl, 3-alkanoyloxyprop-1-enyl or 3-alkanoyloxypropyl group, each having from 1 to 6 carbon atoms, or a 3-hydroxyprop-1-enyl or 3-hydroxypropyl group, and
$R^5$ is a hydrogen atom

or
B)

$R^1$ is a methyl or ethyl group,
$R^2$ is a methoxy, thiomethyl, dimethylamino, monomethylamino, cyano, formyl, acetyl or 1-alkoxyethyl group having from 1 to 6 carbon atoms in the alkoxy radical,
$R^3$ is an alkoxy, alkoxymethyloxy or alkanoyloxyethyloxy group, each having from 1 to 6 carbon atoms in the alkoxy radical, and
$R^4$ is an ethynyl, prop-1-ynyl, alkyl, 3-alkoxyprop-1-ynyl or 3-alkoxypropyl group, each having from 1 to 4 carbon atoms in the alkyl or alkoxy radical, or the grouping $-CH_2Y$ wherein Y is a cyano, azido or alkoxy radical having from 1 to 6 carbon atoms, and
$R^5$ is a hydrogen atom or an alkyl group having from 1 to 4 carbon atoms, or
$R^4$ and $R^5$ together form a tetramethylene bridge

or
C)

$R^1$ is a methyl group,
$R^2$ is a dimethylamino group or a free or ketalised acetyl group,
$R^3$ is an alkoxy group having from 1 to 6 carbon atoms or an alkylthiomethyloxy group having from 1 to 4 carbon atoms in the alkyl radical,
$R^4$ is an alkoxymethyl group having from 1 to 6 carbon atoms in the alkoxy radical, and
$R^5$ is a hydrogen atom.

**2.** Compounds of the general formula I, wherein

$R^1$ is a methyl group,
$R^2$ is a dimethylamino or acetyl group, and also

in A)

$R^3$ is a methoxy group,
$R^4$ is a prop-1-ynyl, ethynyl, 3-hydroxyprop-1-ynyl, 3-hydroxyprop-1(Z)-enyl or 3-hydroxypropyl group and
$R^5$ is a hydrogen atom,

in B)

$R^3$ is a methoxy group,
$R^4$ is a prop-1-ynyl, ethynyl or 3-hydroxypropyl group,
$R^5$ is a hydrogen atom or
$R^4$ and $R^5$ together form a tetramethylene bridge,

in C)

$R^3$ is a methoxy group,
$R^4$ is a methoxymethyl, ethoxymethyl or propoxymethyl group and
$R^5$ is a hydrogen atom.

**3.** 11β-(4-Dimethylaminophenyl)-17β-methoxy-17α-propynyl-13-methyl-gona-4,9-dien-3-one,
11β-(4-acetylphenyl)-17β-methoxy-17α-propynyl-13-methyl-gona-4,9-dien-3-one,
11β-(4-dimethylaminophenyl)-17β-methoxy-17α-ethynyl-13-methyl-gona-4,9-dien-3-one,
11β-(4-acetylphenyl)-17β-methoxy-17α-ethynyl-13-methyl-gona-4,9-dien-3-one,

11β-(4-acetylphenyl)-17α-(3'-hydroxy-1-propynyl)-17β-methoxy-13-methyl-gona-4,9-dien-3-one,
11β-(4-acetylphenyl)-17α-(3'-hydroxy-1'(Z)-propenyl)-17β-methoxy-13-methyl-gona-4,9-dien-3-one, and
11β-(4-acetylphenyl)-17α-(3'-hydroxypropyl)-17β-methoxy-13-methyl-gona-4,9-dien-3-one,
11β-(4-acetylphenyl)-17β-methoxy-17α-methoxymethyl-oestra-4,9-dien-3-one,
11β-(4-dimethylaminophenyl)-17β-methoxy-17α-methoxymethyl-oestra-4,9-dien-3-one,
11β-(4-acetylphenyl)-17α-ethoxymethyl-17β-methoxy-oestra-4,9-dien-3-one,
11β-(4-acetylphenyl)-17β-methoxy-17α-propoxymethyl-oestra-4,9-dien-3-one,
11β-(4-dimethylaminophenyl)-17β-methoxy-17α-propynyl-13-methyl-gona-4,9-dien-3-one,
11β-(4-methoxyphenyl)-17β-methoxy-17α-propynyl-13-methyl-gona-4,9-dien-3-one,
11β-(4-dimethylaminophenyl)-17α-(3'-hydroxy-1'-Z-propenyl)-17β-methoxy-13-methyl-gona-4,9-dien-3-one,
11β-(4-methoxyphenyl)-17β-methoxy-17α-(3'-hydroxy-1'-Z-propenyl)-13-methyl-gona-4,9-dien-3-one,
11β-(4-dimethylaminophenyl)-17α-(3'-hydroxypropyl)-17β-methoxy-13-methyl-gona-4,9-dien-3-one,
11β-(4-methoxyphenyl)-17α-(3'-hydroxypropyl)-17β-methoxy-13-methyl-gona-4,9-dien-3-one,
11β-(4-dimethylaminophenyl)-17α-(3'-hydroxy-1'-propynyl)-17β-methoxy-13-methyl-gona-4,9-dien-3-one,
11β-(4-methoxyphenyl)-17α-(3'-hydroxy-1'-propynyl)-17β-methoxy-13-methyl-gona-4, 9-dien-3-one,
11β-(4-dimethylaminophenyl)-17α-ethynyl-17β-methoxy-13-methyl-gona-4,9-dien-3-one,
11β-(4-methoxyphenyl)-17α-ethynyl-17β-methoxy-13-methyl-gona-4,9-dien-3-one,
11β-(4-methoxyphenyl)-17β-methoxy-17α-methoxymethyl-13-methyl-gona-4,9-dien-3-one.

**4.** Process for the preparation of 11β-aryl-gona-4,9-dienes of the general formula I

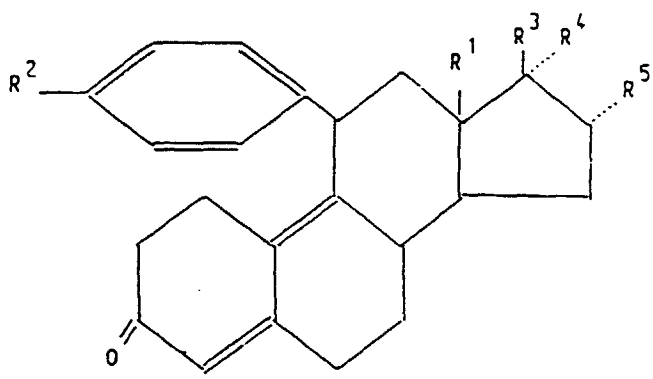

(I)

wherein
$R^1$, $R^2$, $R^3$, $R^4$ and $R^5$ have the meanings given in claim 1,
characterised in that a compound of the general formula II

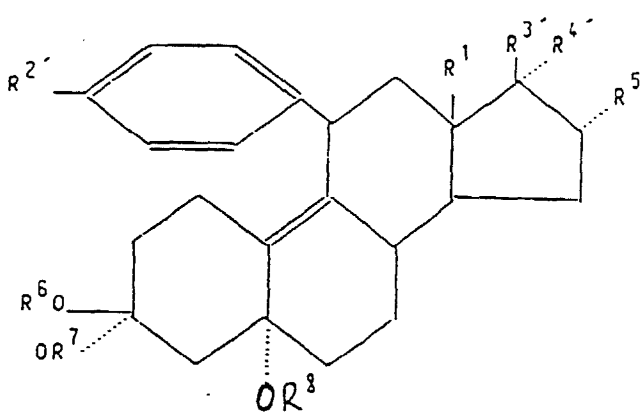

(II)

wherein

$R^1$ and $R^5$ have the meanings given in formula I,
$R^{2'}$, $R^{3'}$ and $R^{4'}$ have the same meanings as $R^2$, $R^3$ and $R^4$ in formula I, it being possible for any keto and/or

hydroxy groups present to be protected,
$R^6$ and $R^7$ are each a methyl or ethyl group or together form an ethylene or 2,2-dialkylpropylene group, especially a 2,2-dimethylpropylene group, and
$R^8$ is a hydrogen atom or an alkyl radical having from 1 to 6 carbon atoms,

is converted by acid treatment in a water-miscible solvent, optionally with heating to 60°-80°C, into a compound of the general formula I.

**5.** Process according to claim 4, characterised in that the acid treatment is carried out with acetic acid, p-toluenesulphonic acid or with mineral acids, such as hydrochloric acid, sulphuric acid, phosphoric acid or perchloric acid in methanol, ethanol or acetone.

**6.** Pharmaceutical preparations, characterised in that they contain at least one compound of the general formula I and a pharmaceutically acceptable carrier.

**7.** The use of compounds of the general formula I in the preparation of medicaments.

**8.** Intermediates of the general formula II

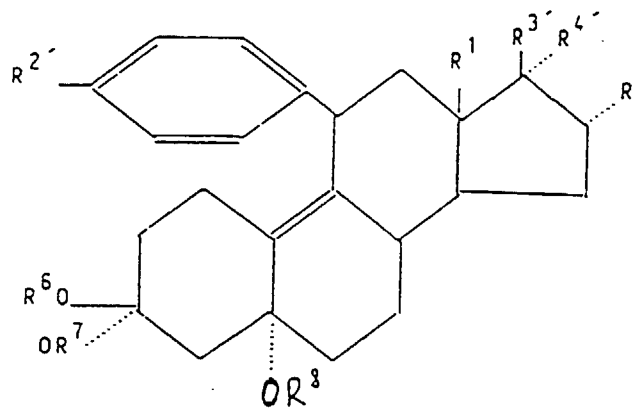

(II)

wherein

$R^1$ and $R^5$ have the meanings given in formula I,
$R^{2'}$, $R^{3'}$ and $R^{4'}$ have the same meanings as $R^2$, $R^3$ and $R^4$ in formula I, it being possible for any keto and/or hydroxy groups present to be protected,
$R^6$ and $R^7$ are each a methyl or ethyl group or together form an ethylene or 2,2-dialkylpropylene group, especially a 2,2-dimethylpropylene group, and
$R^8$ is a hydrogen atom or an alkyl radical having from 1 to 6 carbon atoms.

**Claims for the following Contracting States : ES, GR**

**1.** Process for the preparation of 11β-aryl-gona-4,9-dienes of the general formula I

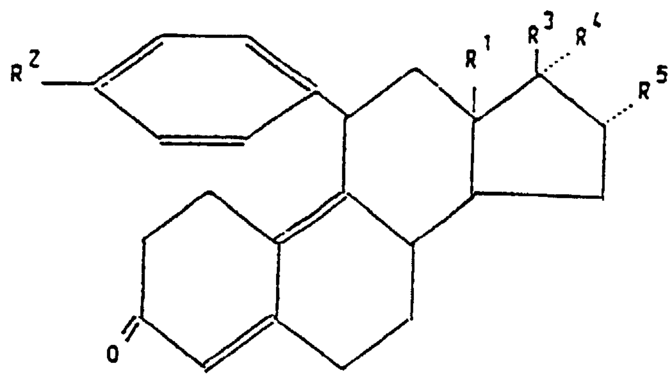

(I)

wherein

A)

$R^1$ is a methyl or ethyl group,
$R^2$ is an alkoxy or alkylthio group, wherein alkyl is to be understood as being alkyl, alkenyl or a corresponding cyclic compound having 1 or 2 or 3 to 7 carbon atoms, respectively, or a dimethylamino, mono-methylamino, cyano, formyl, acetyl or 1-hydroxyethyl group,
$R^3$ is an alkoxymethoxy or alkoxy group each having from 1 to 6 carbon atoms, and
$R^4$ is an ethynyl, prop-1-ynyl, 3-hydroxyprop-1-ynyl, 3-alkanoyloxyprop-1-ynyl, 3-alkanoyloxyprop-1-enyl or 3-alkanoyloxypropyl group, each having from 1 to 6 carbon atoms, or a 3-hydroxyprop-1-enyl or 3-hydroxypropyl group, and
$R^5$ is a hydrogen atom

or
B)

$R^1$ is a methyl or ethyl group,
$R^2$ is a methoxy, thiomethyl, dimethylamino, monomethylamino, cyano, formyl, acetyl or 1-alkoxyethyl group having from 1 to 6 carbon atoms in the alkoxy radical,
$R^3$ is an alkoxy, alkoxymethyloxy or alkanoyloxyethyloxy group, each having from 1 to 6 carbon atoms in the alkoxy radical, and
$R^4$ is an ethynyl, prop-1-ynyl, alkyl, 3-alkoxyprop-1-ynyl or 3-alkoxypropyl group, each having from 1 to 4 carbon atoms in the alkyl or alkoxy radical, or the grouping $-CH_2Y$ wherein Y is a cyano, azido or alkoxy radical having from 1 to 6 carbon atoms, and
$R^5$ is a hydrogen atom or an alkyl group having from 1 to 4 carbon atoms, or
$R^4$ and $R^5$ together form a tetramethylene bridge

or
C)

$R^1$ is a methyl group,
$R^2$ is a dimethylamino group or a free or ketalised acetyl group,
$R^3$ is an alkoxy group having from 1 to 6 carbon atoms or an alkylthiomethyloxy group having from 1 to 4 carbon atoms in the alkyl radical,
$R^4$ is an alkoxymethyl group having from 1 to 6 carbon atoms in the alkoxy radical, and
$R^5$ is a hydrogen atom,

characterised in that a compound of the general formula II

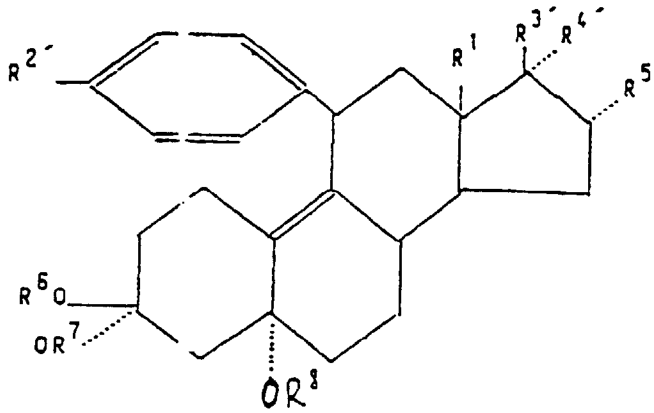

(II)

wherein

$R^1$ and $R^5$ have the meanings given in formula I,
$R^{2'}$, $R^{3'}$ and $R^{4'}$ have the same meanings as $R^2$, $R^3$ and $R^4$ in formula I, it being possible for any keto and/or hydroxy groups present to be protected,
$R^6$ and $R^7$ are each a methyl or ethyl group or together form an ethylene or 2,2-dialkylpropylene group, especially a 2,2-dimethylpropylene group, and
$R^8$ is a hydrogen atom or an alkyl radical having from 1 to 6 carbon atoms,

is converted by acid treatment in a water-miscible solvent, optionally with heating to 60°-80°C, into a compound of the general formula I.

**Revendications**

**Revendications pour les Etats contractants suivants : AT, BE, CH, DE, DK, FR, GB, IT, LI, LU, NL, SE**

**1.** 11β-aryl-gona-4,9-diène de formule générale I:

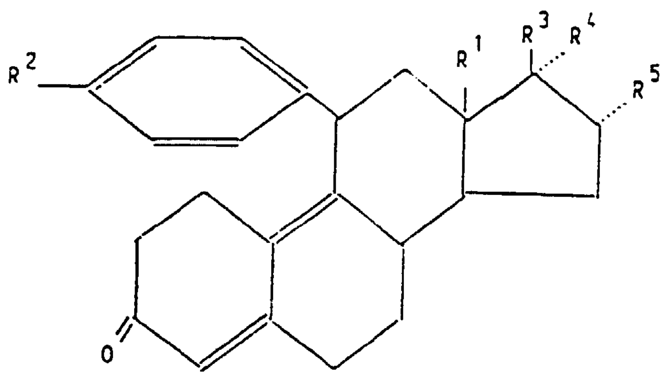

(I)

dans laquelle

A)

$R^1$ est un groupe méthyle ou éthyle,
$R^2$ est un groupe alcoxy, alkylthio, le terme alkyle désignant un groupe alkyle, un groupe alcényle ou une liaison cyclique correspondante avec 1, 2, ou 3 à 7 atomes de carbone, un groupe diméthylamino. monométhylamino, cyano, formyle, acétyle ou 1-hydroxyéthyle,
$R^3$ est un groupe alcoxyméthoxy ou alcoxy avec respectivement 1 à 6 atomes de carbone, et

$R^4$ est un groupe éthinyle, prop-1-inyle, 3-hydroxyprop-1-inyle, 3-alcanoyloxyprop-1-inyle, 3-alcanoyloxyprop-1-ényle, 3-alcanoyloxypropyle avec respectivement 1 à 6 atomes de carbone, 3-hydroxyprop-1-ényle et 3-hydroxypropyle, et
$R^5$ est un atome d'hydrogène

ou
B)

$R^1$ est un groupe méthyle ou éthyle,
$R^2$ est un groupe méthoxy, thiométhyle, diméthylamino, monométhylamino, cyano, formyle, acétyle ou 1-alcoxyéthyle avec 1 à 6 atomes de carbone dans le radical alcoxy,
$R^3$ est un groupe alcoxy, alcoxyméthyloxy, alcanoyloxyéthyloxy avec respectivement 1 à 6 atomes de carbone dans le radical alcoxy, et
$R^4$ est un groupe éthinyle, prop-1-inyle, alkyle, 3-alcoxyprop-1-inyle, 3-alcoxypropyle avec respectivement 1 à 4 atomes de carbone dans le radical alkyle ou alcoxy ou le groupe $-CH_2Y$, dans lequel Y est un radical cyano, azido ou alcoxy avec 1 à 6 atomes de carbone, et
$R^5$ est un atome d'hydrogène ou un groupe alkyle avec 1 à 4 atomes de carbone, ou
$R^4$ et $R^5$ forment ensemble un pont tétraméthylène,

ou encore
C)

$R^1$ est un groupe méthyle,
$R^2$ est un groupe diméthylamino, un groupe acétyle libre ou cétalisé,
$R^3$ est un groupe alcoxy avec 1 à 6 atomes de carbone ou un groupe alkylthiométhyloxy avec 1 à 4 atomes de carbone dans le radical alkyle,
$R^4$ est un groupe alcoxyméthyle avec 1 à 6 atomes de carbone dans le radical alcoxy, et
$R^5$ est un atome d'hydrogène.

**2.** Composés de formule générale I,
dans lesquels

$R^1$ est un groupe méthyle,
$R^2$ est un groupe diméthylamino ou acétyle et, en outre,

dans A),

$R^3$ est un groupe méthoxy,
$R^4$ est un groupe prop-1-inyle, éthinyle, 3-hydroxy-prop-1-inyle, 3-hydroxy-prop-1(Z)-ényle, 3-hydroxypropyle, et
$R^5$ est un atome d'hydrogène,

dans B),

$R^3$ est un groupe méthoxy,
$R^4$ est un groupe prop-1-inyle, éthinyle ou 3-hydroxypropyle,
$R^5$ est un atome d'hydrogène, ou
$R^4$ et $R^5$ forment ensemble un pont tétraméthylène,

dans C),

$R^3$ est un groupe méthoxy,
$R^4$ est un groupe méthoxyméthyle, éthoxyméthyle ou propoxyméthyle, et
$R^5$ est un atome d'hydrogène.

**3.** 11β-(4-diméthylaminophényl)-17β-méthoxy-17α-propinyl-13-méthyl-gona-4,9-dién-3 -one,
11β-(4-acétylphényl)-17β-méthoxy-17α-propinyl-13-méthyl-gona-4,9-dién-3-one,
11β-(4-diméthylaminophényl)-17β-méthoxy-17α-éthinyl-13-méthyl-gona-4,9-dién-3-one,
11β-(4-acétylphényl)-17β-méthoxy-17α-éthinyl-13-méthyl-gona-4,9-dién-3-one,

11β-(4-acétylphényl)-17α-(3'-hydroxy-1-propinyl)-17β-méthoxy-13-méthyl-gona-4,9-dién-3 -one,
11β-(4-acétylphényl)-17α-(3'-hydroxy-1'(Z)-propényl)-17β-méthoxy-13-méthyl-gona-4,9-dién-3-one,
11β-(4-acétylphényl)-17α-(3'-hydroxypropyl)-17β-méthoxy-13-méthyl-gona-4,9-dién-3-one,
11β-(4-acétylphényl)-17β-méthoxy-17α-méthoxyméthyl-oestra-4,9-dién-3-one,
11β-(4-diméthylaminophényl)-17β-méthoxy-17α-méthoxyméthyl-oestra-4,9-dién-3-one,
11β-(4-acétylphényl)-17α-éthoxyméthyl-17β-méthoxy-oestra-4,9-dién-3-one,
11β-(4-acétylphényl)-17β-méthoxy-17α-propoxyméthyl-oestra-4,9-dién-3-one,
11β-(4-diméthylaminophényl)-17β-méthoxy-17α-propinyl-13-méthyl-gona-4,9-dién-3-one,
11β-(4-méthoxyphényl)-17β-méthoxy-17α-propinyl-13-méthyl-gona-4,9-dién-3-one,
11β-(4-diméthylaminophényl)-17α-(3'-hydroxy-1'-Z-propényl)-17β-méthoxy-13-méthyl-gona-4,9-dién-3-one,
11β-(4-méthoxyphényl)-17β-méthoxy-17α-(3'-hydroxy-1'-Z-propényl)-13-méthyl-gona-4,9-dién-3-one,
11β-(4-diméthylaminophényl)-17α-(3'-hydroxypropyl)-17β-méthoxy-13-méthyl-gona-4,9-dién-3-one,
11β-(4-méthoxyphényl)-17α-(3'-hydroxypropyl)-17β-méthoxy-13-méthyl-gona-4,9-dién-3-one,
11β-(4-diméthylaminophényl)-17α-(3'-hydroxy-1'-propinyl)-17β-méthoxy-13-méthyl-gona-4,9-dién-3-one,
11β-(4-méthoxyphényl)-17α-(3'-hydroxy-1'-propinyl)-17β-méthoxy-13-méthyl-gona-4,9-dién-3-one,
11β-(4-diméthylaminophényl)-17α-éthinyl-17β-méthoxy-13-méthyl-gona-4,9-dién-3-one,
11β-(4-méthoxyphényl)-17α-éthinyl-17β-méthoxy-13-méthyl-gona-4,9-dién-3-one,
11β-(4-méthoxyphényl)-17β-méthoxy-17α-méthoxyméthyl-13-méthyl-gona-4,9-dién-3-one.

**4.** Procédé de préparation de 11β-aryl-gona-4,9-diènes de formule générale I:

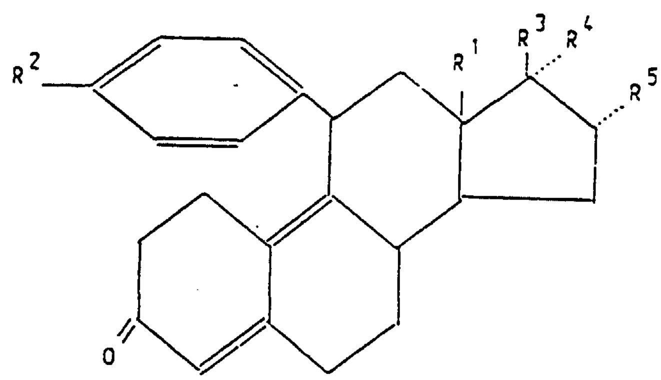

(I)

dans laquelle
$R^1$, $R^2$, $R^3$, $R^4$ et $R^5$ ont la signification indiquée dans la revendication 1. caractérisé en ce qu'un composé de formule générale II :

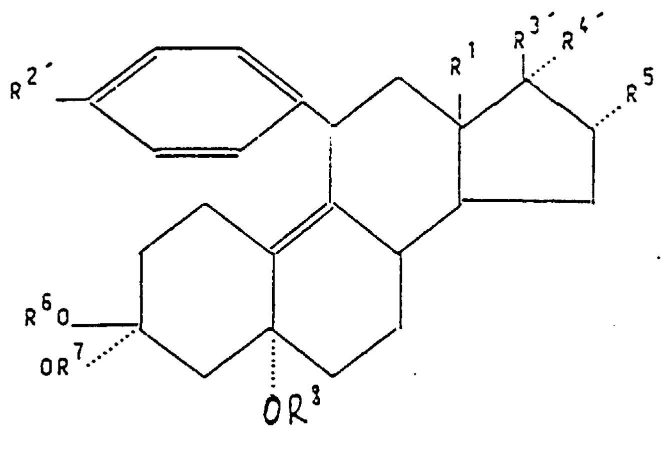

(II)

dans laquelle

$R^1$ et $R^5$ ont la signification indiquée dans la formule I,
$R^{2'}$, $R^{3'}$ et $R^{4'}$ ont la même signification que $R^2$, $R^3$ et $R^4$ dans la formule I, les groupes céto et/ou hydroxy

éventuellement présents pouvant être protégés,
$R^6$ et $R^7$ sont chacun un groupe méthyle ou éthyle ou forment ensemble un groupe éthylène ou 2,2-dialkylpropylène, en particulier un groupe 2,2-diméthylpropylène, et
$R^8$ est un atome d'hydrogène ou un radical alkyle avec 1 à 6 atomes de carbone,

est converti en un composé de formule générale I par traitement acide dans un solvant miscible à l'eau, éventuellement par chauffage à 60-80°C.

**5.** Procédé selon la revendication 4, caractérisé en ce que le traitement acide est réalisé avec de l'acide acétique, de l'acide p-toluènesulfonique ou avec des acides minéraux tels que l'acide chlorhydrique, l'acide sulfurique, l'acide phosphorique ou l'acide perchlorique dans du méthanol, de l'éthanol ou de l'acétone.

**6.** Préparations pharmaceutiques, caractérisées en ce qu'elles contiennent au moins un composé de formule générale I, ainsi qu'un support pharmaceutiquement compatible.

**7.** Utilisation de composés de formule générale I pour préparer des médicaments.

**8.** Composés intermédiaires de formule générale II :

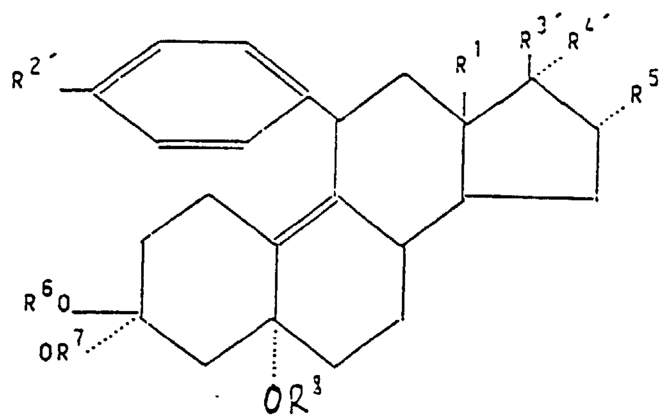

(II)

dans laquelle

$R^1$ et $R^5$ ont la signification indiquée dans la formule I,
$R^{2'}$, $R^{3'}$ et $R^{4'}$ ont la même signification que $R^2$, $R^3$ et $R^4$ dans la formule I. les groupes céto et/ou hydroxy éventuellement présents pouvant être protégés,
$R^6$ et $R^7$ sont chacun un groupe méthyle ou éthyle ou forment conjointement un groupe éthylène ou 2,2-dialkylpropylène, en particulier un groupe 2,2-diméthylpropylène, et
$R^8$ est un atome d'hydrogène ou un radical alkyle avec 1 à 6 atomes de carbone.

**Revendications pour les Etats contractants suivants : ES, GR**

**1.** Procédé de préparation de 11β-aryl-gona-4,9-diènes de formule générale I:

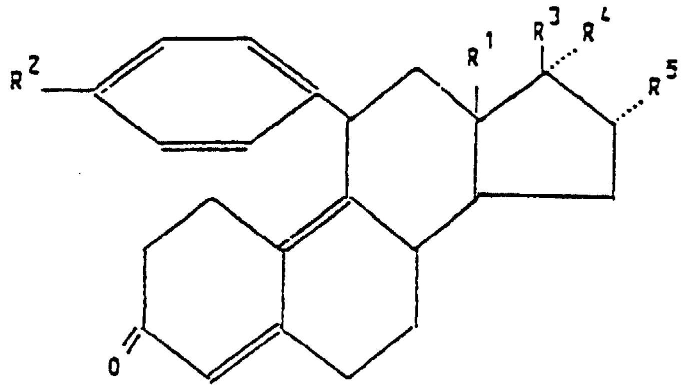

(I)

dans laquelle

A)

$R^1$ est un groupe méthyle ou éthyle,

$R^2$ est un groupe alcoxy, alkylthio, le terme alkyle désignant un groupe alkyle, un groupe alcényle ou une liaison cyclique correspondante avec 1, 2, ou 3 à 7 atomes de carbone, un groupe diméthylamino, monométhylamino, cyano, formyle, acétyle ou 1-hydroxyéthyle,

$R^3$ est un groupe alcoxyméthoxy ou alcoxy avec respectivement 1 à 6 atomes de carbone, et

$R^4$ est un groupe éthinyle, prop-1-inyle, 3-hydroxyprop-1-inyle, 3-alcanoyloxyprop-1-inyle, 3-alcanoyloxyprop-1-ényle, 3-alcanoyloxypropyle avec respectivement 1 à 6 atomes de carbone, 3-hydroxyprop-1-ényle et 3-hydroxypropyle, et

$R^5$ est un atome d'hydrogène

ou

B)

$R^1$ est un groupe méthyle ou éthyle,

$R^2$ est un groupe méthoxy, thiométhyle, diméthylamino, monométhylamino, cyano, formyle, acétyle ou 1-alcoxyéthyle avec 1 à 6 atomes de carbone dans le radical alcoxy,

$R^3$ est un groupe alcoxy, alcoxyméthyloxy, alcanoyloxyéthyloxy avec respectivement 1 à 6 atomes de carbone dans le radical alcoxy, et

$R^4$ est un groupe éthinyle, prop-1-inyle, alkyle, 3-alcoxyprop-1-inyle, 3-alcoxypropyle avec respectivement 1 à 4 atomes de carbone dans le radical alkyle ou alcoxy ou bien le groupe $-CH_2Y$, dans lequel Y est un radical cyano, azido ou alcoxy avec 1 à 6 atomes de carbone, et

$R^5$ est un atome d'hydrogène ou un groupe alkyle avec 1 à 4 atomes de carbone, ou

$R^4$ et $R^5$ forment ensemble un pont tétraméthylène,

ou

C)

$R^1$ est un groupe méthyle,

$R^2$ est un groupe diméthylamino, un groupe acétyle libre ou cétalisé,

$R^3$ est un groupe alcoxy avec 1 à 6 atomes de carbone ou un groupe alkylthiométhyloxy avec 1 à 4 atomes de carbone dans le radical alkyle,

$R^4$ est un groupe alcoxyméthyle avec 1 à 6 atomes de carbone dans le radical alcoxy, et

$R^5$ est un atome d'hydrogène,

caractérisé en ce qu'un composé de formule générale II :

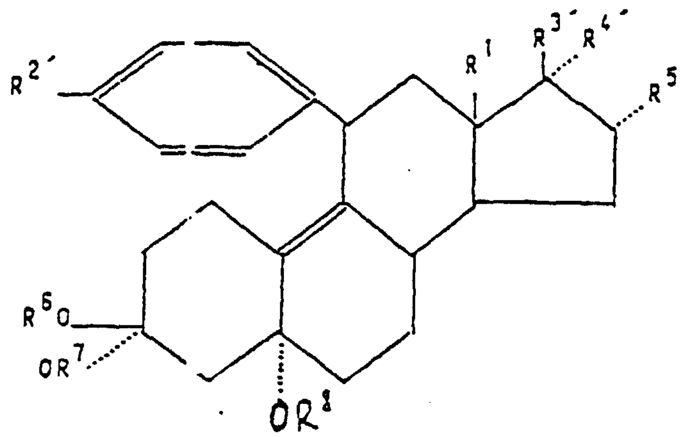

(II)

dans laquelle

$R^1$ et $R^5$ ont la signification indiquée dans la formule I,
$R^{2'}$, $R^{3'}$ et $R^{4'}$ ont la même signification que $R^2$, $R^3$ et $R^4$ dans la formule I, les groupes céto et/ou hydroxy éventuellement présents pouvant être protégés,
$R^6$ et $R^7$ sont chacun un groupe méthyle ou éthyle ou forment ensemble un groupe éthylène ou 2,2-dialkylpropylène, en particulier un groupe 2,2-diméthylpropylène, et
$R^8$ est un atome d'hydrogène ou un radical alkyle avec 1 à 6 atomes de carbone,

est converti en un composé de formule générale I par traitement acide dans un solvant miscible à l'eau, éventuellement par chauffage à 60-80°C.